# EUROPEAN PATENT SPECIFICATION

(11) **EP 2 861 988 B1**
(45) Date of publication and mention of the grant of the patent: **22.05.2019**
(21) Application number: 13737006.0
(22) Date of filing: 14.06.2013
(51) Int. Cl.: G01N 33/50

(54) **GENOME-WIDE METHOD OF ASSESSING INTERACTIONS BETWEEN CHEMICAL ENTITIES AND THEIR TARGET MOLECULES**
GENOMWEITES VERFAHREN ZUR BESTIMMUNG DER INTERAKTION VON CHEMISCHEN ENTITÄTEN UND DEREN ZIELMOLEKÜLEN
PROCÉDÉ À L'ÉCHELLE DU GÉNOME D'ÉVALUATION D'INTERACTIONS ENTRE DES ENTITÉS CHIMIQUES ET LEURS MOLÉCULES CIBLES

(30) Priority: 14.06.2012 US 201261659750 P; 06.07.2012 US 201261668718 P; 14.09.2012 US 201261701434 P; 15.03.2013 US 201361800060 P
(43) Date of publication of application: 22.04.2015
(73) Proprietor: The Whitehead Institute for Biomedical Research, Cambridge, MA 02142 (US); Dana-Farber Cancer Institute, Inc., Boston, MA 02115-5450 (US)
(72) Inventor: YOUNG, Richard, A., Weston, MA 02493 (US); BRADNER, James, E., Weston, MA 02493 (US); RAHL, Peter, B., Natick, MA 01760 (US); ANDERS, Lars, Boston, MA 02215 (US); MARINEAU, Jason, J., Franklin, MA 02038 (US); QI, Jun, Sharon, MA 02067 (US); GUENTHER, Matthew, G., Boston, MA 02108 (US)
(74) Representative: Lock, Graham James
(86) International application number: PCT/US2013/045929
(87) International publication number: WO 2013/188789

(56) References cited:
- WO-A2-01/16378
- WO-A2-2005/054461
- WO-A2-2007/030678
- US-A1- 2004 058 356
- PANAGIS FILIPPAKOPOULOS ET AL: "Selective inhibition of BET bromodomains", NATURE: INTERNATIONAL WEEKLY JOURNAL OF SCIENCE (AND SUPPLEMENTARY INFORMATION), NATURE PUBLISHING GROUP, UNITED KINGDOM, vol. 468, 23 December 2010 (2010-12-23), pages 1067-1073, XP007919792, ISSN: 0028-0836, DOI: 10.1038/NATURE09504 cited in the application
- JAKEE DELMORE ET AL: "BET Bromodomain Inhibition as a Therapeutic Strategy to Target c-Myc", CELL, CELL PRESS, US, vol. 146, no. 6, 15 August 2011 (2011-08-15), pages 904-917, XP028295705, ISSN: 0092-8674, DOI: 10.1016/J.CELL.2011.08.017 [retrieved on 2011-08-18] cited in the application
- Angela R. Wu ET AL: "High throughput automated chromatin immunoprecipitation as a platform for drug screening and antibody validation", Lab on a Chip, vol. 12, no. 12, 1 January 2012 (2012-01-01), page 2190, XP055261502, GB ISSN: 1473-0197, DOI: 10.1039/c2lc21290k
- Benjamin Garcia: "Reverse ChIP for Anti-Cancer Drug Screening and Epigenetic Oncology - Research at Princeton", , 9 December 2011 (2011-12-09), XP055261506, Retrieved from the Internet: URL:http://www.princeton.edu/research/news /features/a/?id=6231 [retrieved on 2016-03-30]
- Ana Ricobaraza ET AL: "Phenylbutyrate Ameliorates Cognitive Deficit and Reduces Tau Pathology in an Alzheimer's Disease Mouse Model", Neuropsychopharmacology, vol. 34, no. 7, 14 January 2009 (2009-01-14), pages 1721-1732, XP055019203, ISSN: 0893-133X, DOI: 10.1038/npp.2008.229
- M. V. Simonini ET AL: "From The Cover: The benzamide MS-275 is a potent, long-lasting brain region-selective inhibitor of histone deacetylases", Proceedings of the National Academy of Sciences, vol. 103, no. 5, 31 January 2006 (2006-01-31), pages 1587-1592, XP055261527, US ISSN: 0027-8424, DOI: 10.1073/pnas.0510341103
- THOMAS ELIZABETH A ET AL: "The HDAC inhibitor 4b ameliorates the disease phenotype and transcriptional abnormalities in Huntington's disease transgenic mice", PROCEEDINGS OF THE NATIONAL ACADEMY OF SCIENCES, NATIONAL ACADEMY OF SCIENCES, US, vol. 105, no. 40, 7 October 2008 (2008-10-07), pages 15564-15569, XP002574044, ISSN: 0027-8424, DOI: 10.1073/PNAS.0804249105 [retrieved on 2008-09-30]
- John Comley: "EPIGENETICS an emerging target class for drug screening", Drug Discovery World, 1 January 2011 (2011-01-01), pages 40-55, XP055261515, Retrieved from the Internet: URL:http://www.ddw-online.com/media/32/222 7/epigenetics.pdf [retrieved on 2016-03-30]
- RICKE D O ET AL: "Genomic approaches to drug discovery", CURRENT OPINION IN CHEMICAL BIOLOGY, CURRENT BIOLOGY LTD, LONDON, GB, vol. 10, no. 4, 1 August 2006 (2006-08-01) , pages 303-308, XP028014471, ISSN: 1367-5931, DOI: 10.1016/J.CBPA.2006.06.024 [retrieved on 2006-08-01]
- SHU-YUN TUNG ET AL: "Chromatin affinity-precipitation using a small metabolic molecule: its application to analysis of-acetyl-ADP-ribose", CMLS CELLULAR AND MOLECULAR LIFE SCIENCES, BIRKHÄUSER-VERLAG, BA, vol. 69, no. 4, 28 July 2011 (2011-07-28), pages 641-650, XP035006852, ISSN: 1420-9071, DOI: 10.1007/S00018-011-0771-X

## Description

### RELATED APPLICATIONS

This application claims the benefit of U.S. Provisional Application No. 61/659,750, filed on June 14, 2012 and U.S. Provisional Application No. 61/668,718, filed on July 6, 2012 and U.S. Provisional Application No. 61/701,434, filed on September 14, 2012 and U.S. Provisional Application No. 61/800,060, filed on March 15, 2013.

### GOVERNMENT SUPPORT

This invention was made with government support under RO1-HG002668 from the National Institutes of Health and PF-11-042-01-DMC from the American Cancer Society. The government has certain rights in the invention.

### BACKGROUND OF THE INVENTION

Recent technological advances have allowed the genome-wide study of gene regulatory networks by localizing proteins to specific regions of DNA or associated chromatin. Deep sequencing of DNA associated with proteins of interest, pulled-down using directed antibodies, provides high-resolution spatial maps of genomic organization. Termed chromatin immunoprecipitation with deep sequencing (ChIP-seq), biologists now possess a new lens through which they can understand cellular and disease biology. However, there are important limitations to this technology. The procedure requires the existence of validated, specific immunoglobulins and only individual protein/DNA complexes may be isolated. The complexity of this experiment has limited the use of ChIP-seq to understand and characterize the effects of chromatin-directed small molecules.

### SUMMARY OF THE INVENTION

Described herein is a genome-wide assessment of interactions between chemical entities and their target molecules, referred to as Chem-seq technology.

Accordingly, in one aspect, the invention is directed to a method of mapping, across a genome, interaction of a drug directly or indirectly with one or more regions of the genome as defined in the appended claims.

In another aspect, the invention is directed to a method of identifying one or more sites on a genome with which a drug interacts directly or indirectly as defined in the appended claims.

In another aspect, the invention is directed to a method of identifying a signature of a disease across a genome as defined in the appended claims.

In yet another aspect, the invention is directed to a method of obtaining a genetic signature of a drug's interaction with the genome as defined in the appended claims.

The disclosure describes features of a kit, the kit comprising one or more of (e.g., each of);
(a) a cell or cell lysate or nuclear extract that comprises chromatin or reagent useful for making of a cell lysate or nuclear extract that comprises chromatin;
(b) a drug or drug candidate, wherein the drug or drug candidate is linked to a detectable label such as biotin or coupled to a solid support (e.g., directly or indirectly);
(c) a factor that interacts with the label (e.g., wherein the factor is attached to a solid support such as a bead) (exemplary factors include an antibody (e.g, attached to a solid support));
(d) optionally, a reagent, for example, a primer or oligo such as a PCR oligo for sequencing one or more sequences identified by the method of the instructions from the kit; and
instructions to maintain the drug or drug candidate and cell or cell lysate or nuclear extract that comprises chromatin under conditions in which the drug or drug candidate can interact with the one or more factors that associate with the genome, and the one or more factors that associate with the genome are covalently linked to the genome of the cell; instructions to fragment the genome of the cell or cell lysate or nuclear extract that comprises chromatin thereby producing a mixture comprising genome fragments, wherein one or more of the genome fragments are associated with the one or more of the factors which interact with the drug or drug candidate; and instructions to determine all or a portion of the sequence of the one or more genome fragments that are associated with the one or more factors which interact with the drug or drug candidate, thereby mapping, across the genome, the interaction of the drug or drug candidate with one or more factors that associate with the genome.

### BRIEF DESCRIPTION OF THE DRAWINGS

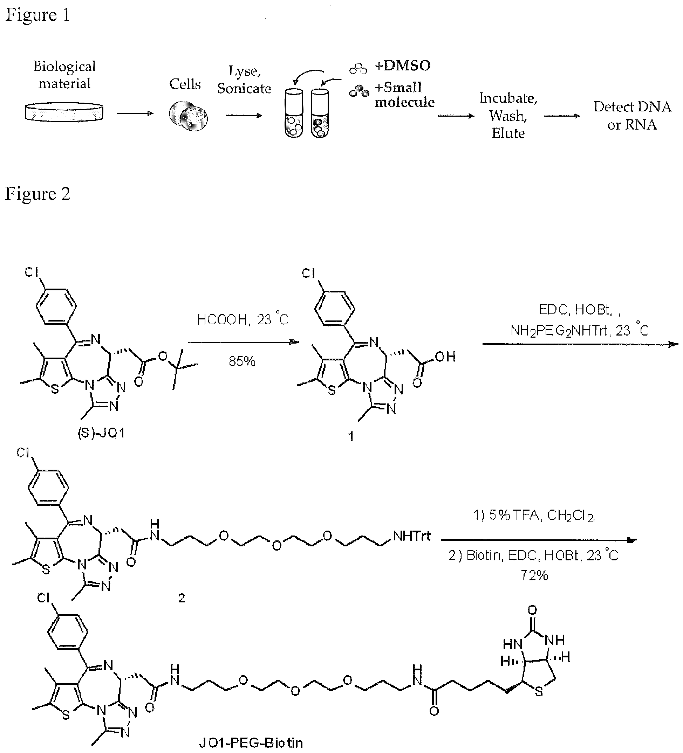

Fig. 1 is a schematic of the Chem-Seq approach.
Fig. 2 shows synthesis of JQ1-PEG-Biotin.

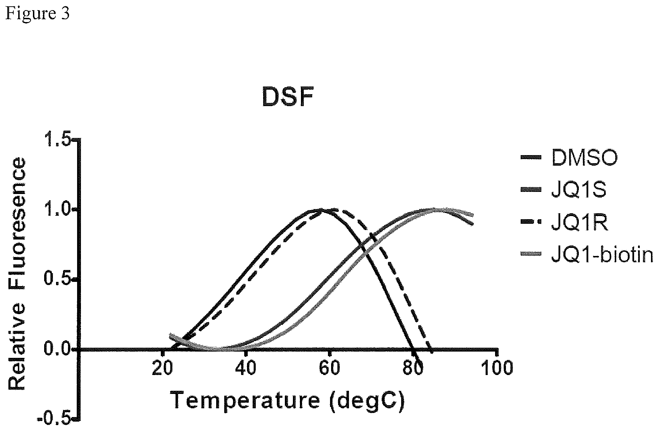

Fig. 3 shows the results of differential scanning fluorimetry (DSF) of JQ1-PEG-Biotin.

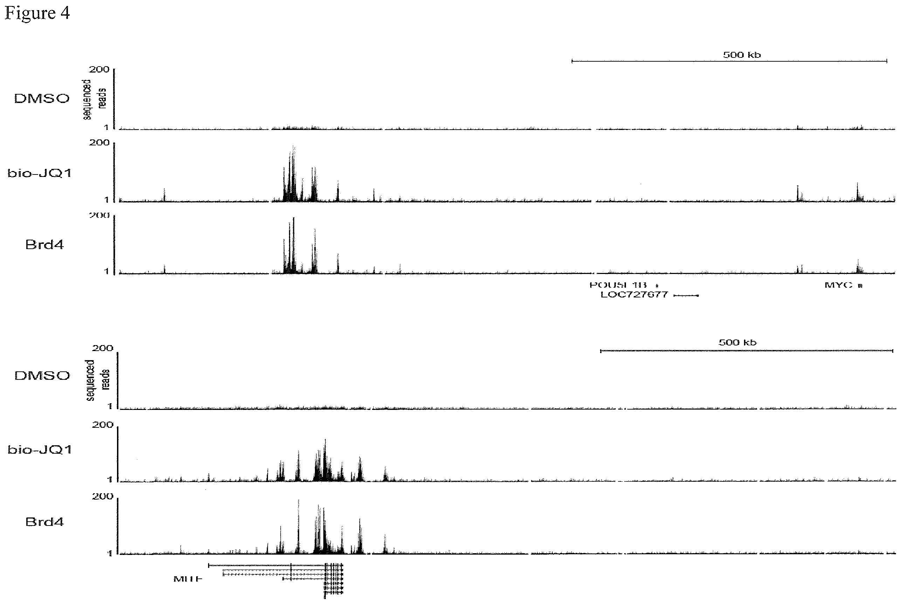

Fig. 4 shows chem-seq analysis of the association of bio-JQ1 and genomic regions near the MYC and MITF loci. Enrichment of sequenced reads for DMSO (negative control), bio-JQ1, and Brd4 (a protein target of JQ1) show a high degree of overlap for bio-JQ1 and Brd4 at regions across the genome, including at regions near the MYC (A) and MITF (B) genes.

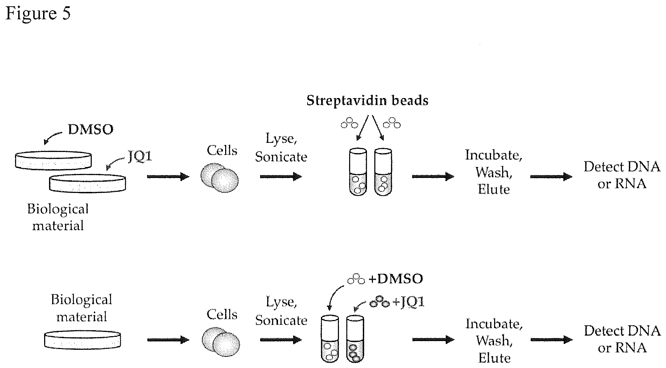

Fig. 5 is a schematic representation of the Chem-Seq in vivo and in vitro approach. In vivo Chem-Seq allows analysis of drug-genome binding in living cells. In vitro Chem-Seq is an alternative Chem-Seq approach for determining the genomic binding sites for drugs or chemical entities that are not cell permeable.

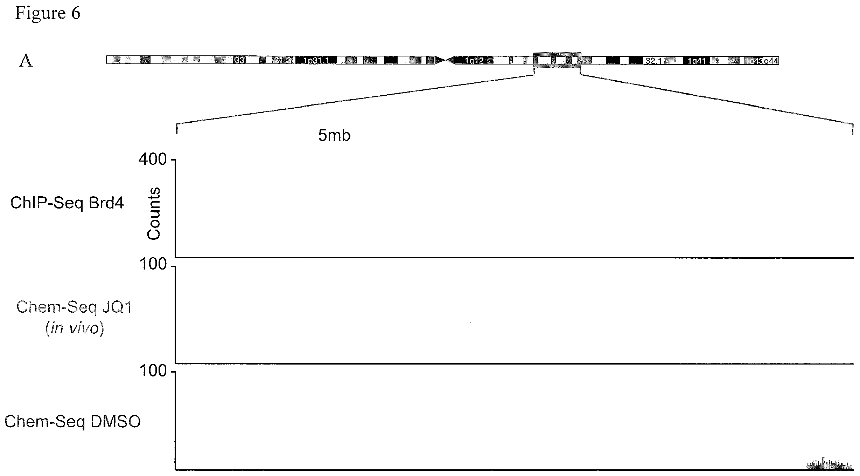

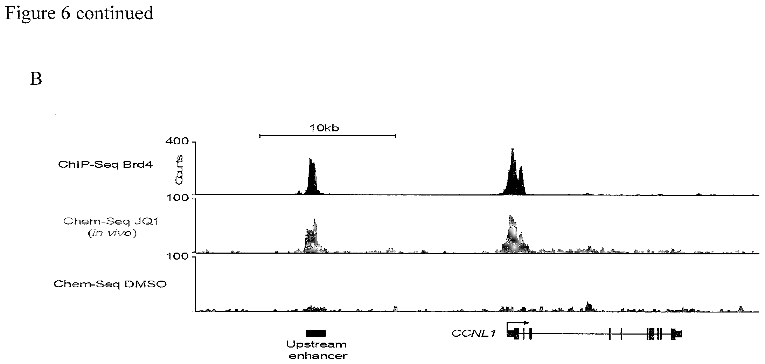

Fig. 6 shows Chem-Seq in vivo analysis of the association of bio-JQ1 and genomic regions in cancer cells. In vivo Chem-Seq revealed genomic sites bound by the drug JQ1 in living cells. For comparison, a genomic location analysis (ChIP-Seq) of the JQ1 target protein, BRD4, is shown. Note the degree of overlap between the sites bound in vivo by JQ1 and those occupied by BRD4. A: Visualization of JQ1 occupied sites on a region of chromosome 1. B: JQ1 in vivo Chem-Seq analysis of the CCNL1 gene locus.

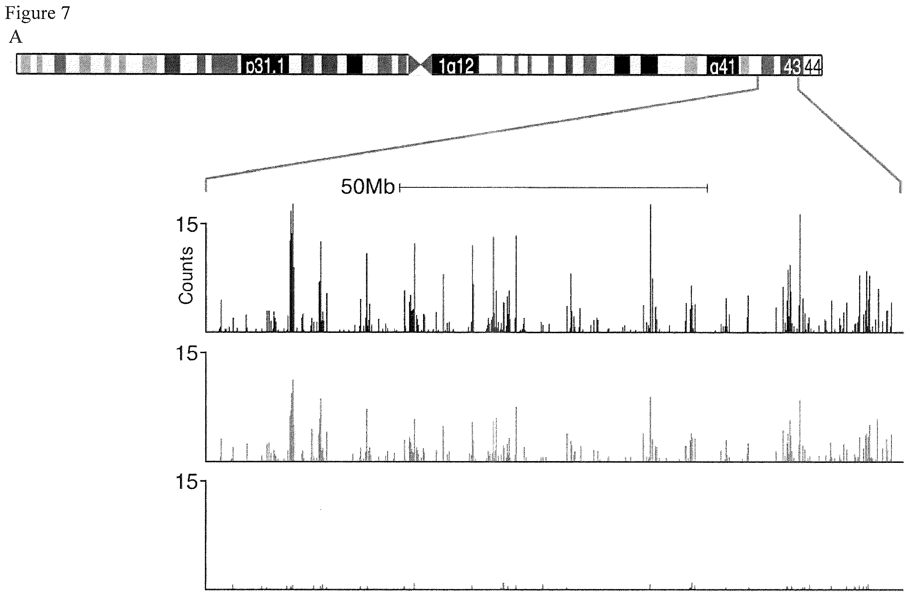

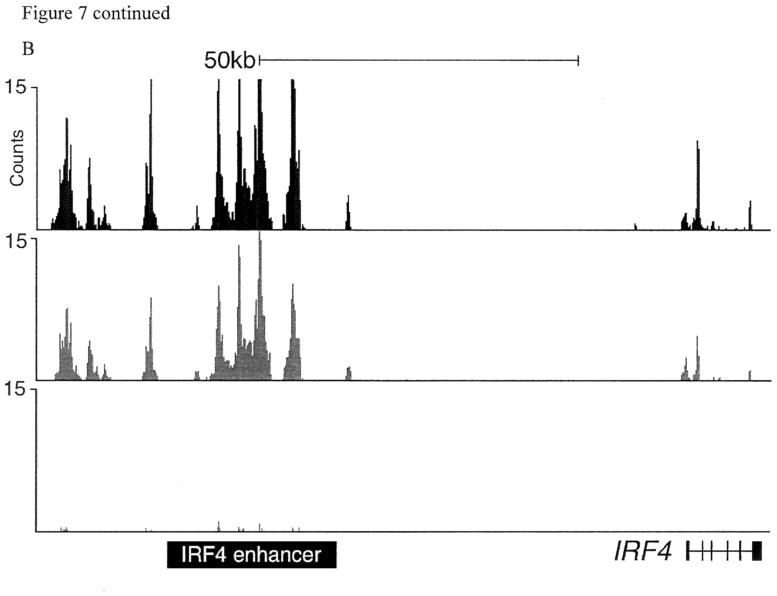

Fig. 7 illustrates Chem-seq in vitro analysis of bio-JQ1 binding to genomic regions in cancer cells. In vitro Chem-Seq reveals genomic sites bound by the drug JQ1 (red). For comparison, a genomic location analysis (ChIP-Seq) of the JQ1 target protein, BRD4 (black) is shown. Note the degree of overlap between the sites bound in vitro by JQ1 and those occupied by BRD4. A: Visualization of JQ1 occupied sites on a region of chromosome 1. B: JQ1 In vitro Chem-Seq analysis of the IRF4 gene locus which illustrates that *in vitro* Chem-Seq produces the same result as *in vivo* Chem-Seq: JQ1 target sites overlap BRD4-bound sites.

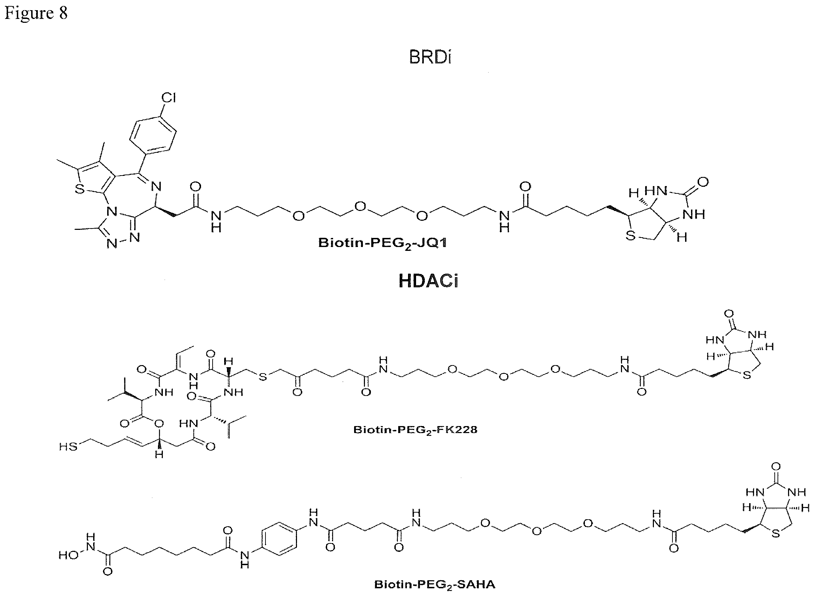

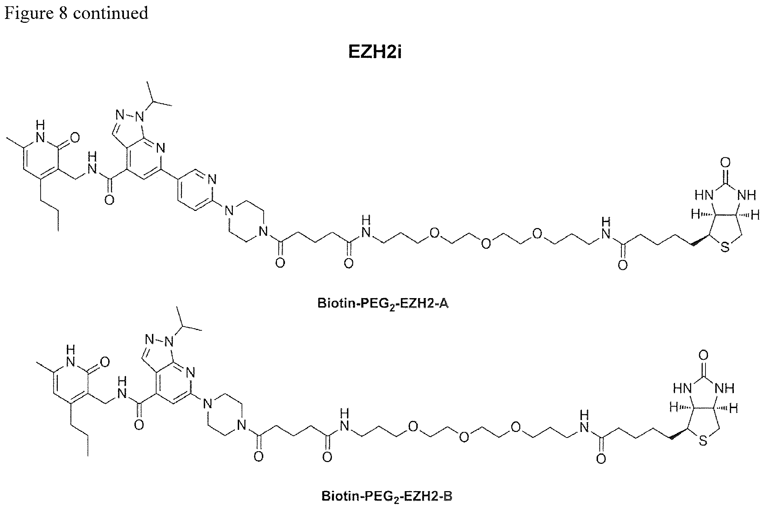

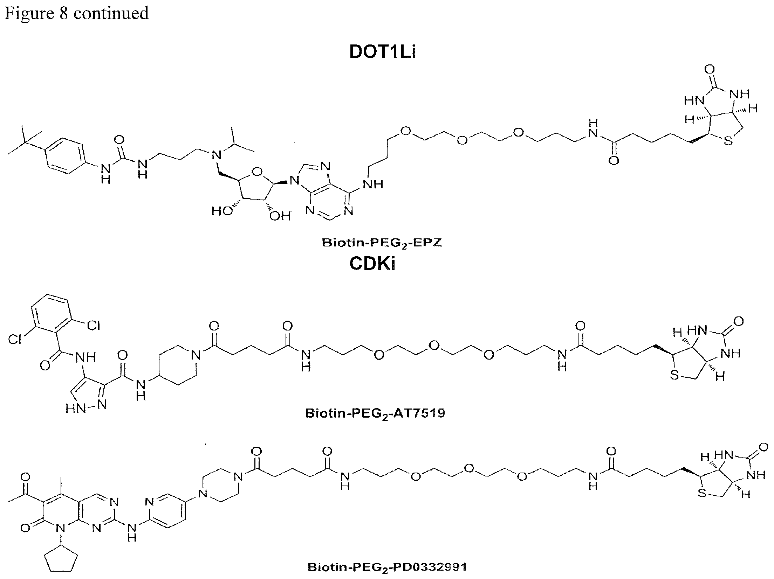

Figure 8 show examples of biotinylated compounds for analysis by the Chem-Seq methods described herein.

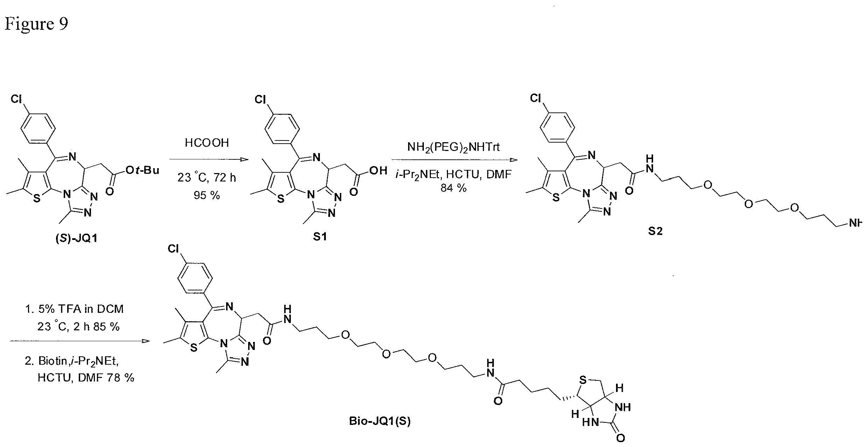

Figure 9. shows synthesis of compound Bio-JQ1(S).

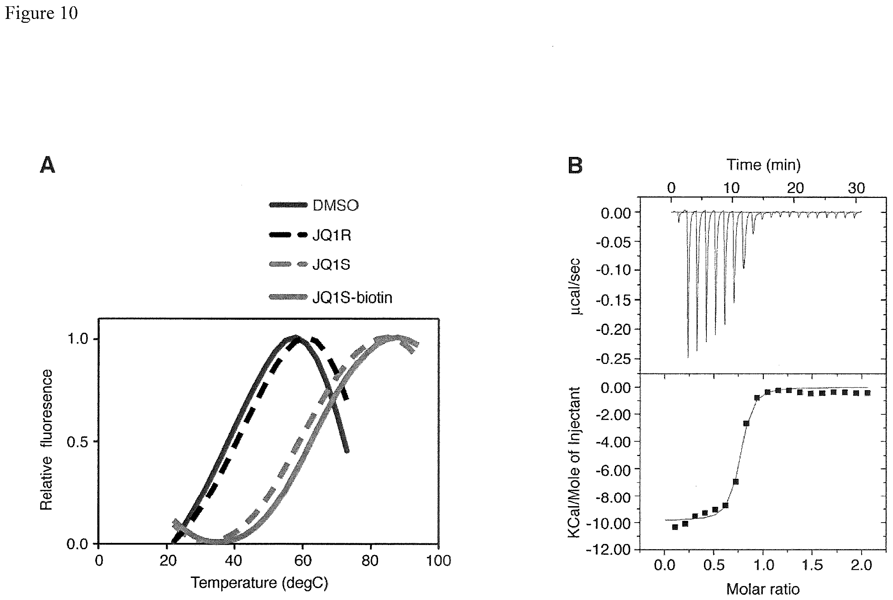

Fig. 10. Biochemical characterization of biotinylated JQ1 (bio-JQ1). A. Results of differential scanning fluorimetry using the purified bromodomain of BRD4, BRD4 (1) and DMSO, JQ1, bio-JQ1 and JQ1R. B. Results of isothermal titration calorimetry and determination of the dissociation constant for biotinylated JQ1 (nio-JQ1)

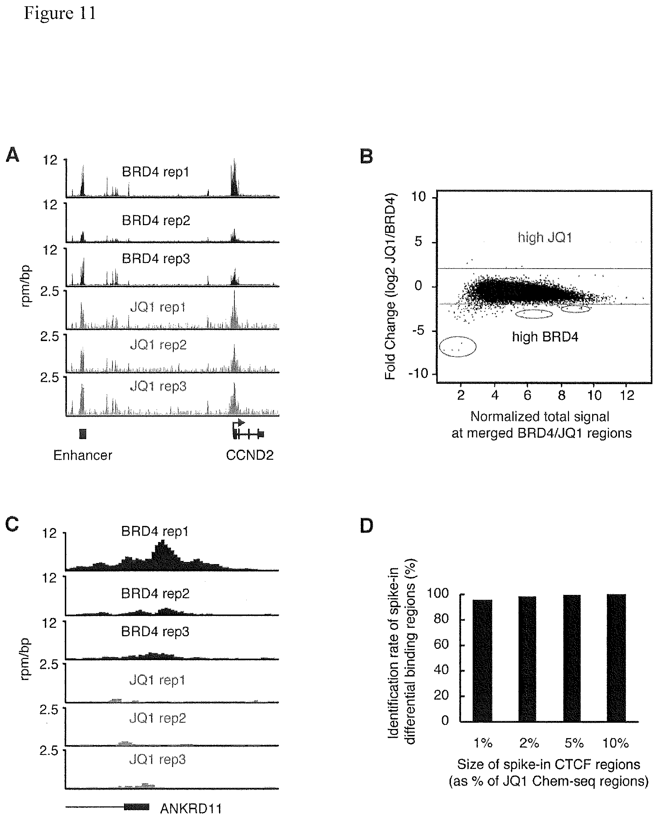

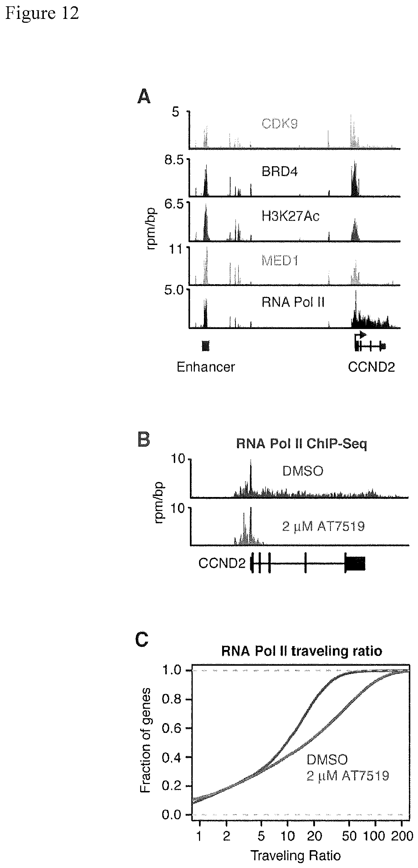

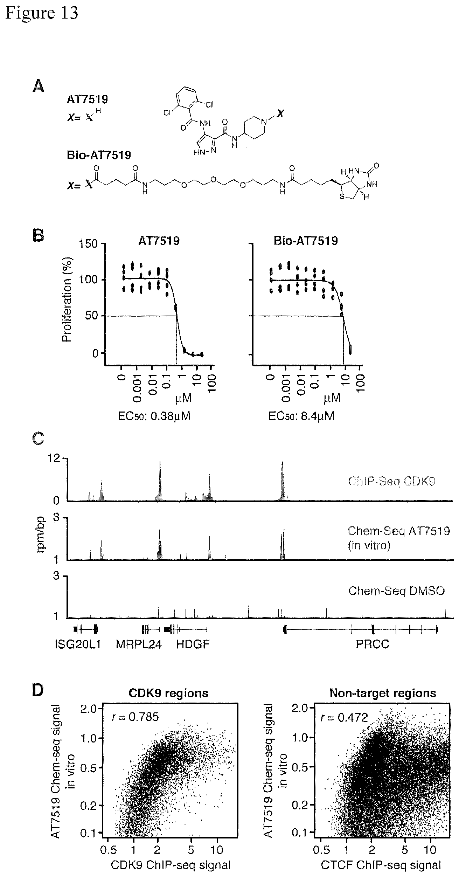

Fig. 11. Specificity analysis based on overlap between Chem-Seq and ChIP-Seq data. A. Occupancy of replicates of BRD4 by ChIP-Seq and JQ1 by Chem-Seq at the CCND2 gene in MM1.S cells. B. Fold change of normalized JQ1 over BRD4 signal at 25060 merged BRD4/JQ1 regions. Differential binding analysis revealed only 10 statistical significant regions out of 25060 merged BRD4/JQ1 regions showing differential binding (red dots, FDR<=0.25, highlighted in red circles). C. Occupancy of replicates of BRD4 by ChIP-Seq and JQ1 by Chem-Seq at one of the 10 differential binding sites in MM1.S cells. D. Identification rate (%) of simulated JQ1 peaks is shown when variable numbers of CTCF peaks were introduced as differential binding sites. In the simulated JQ1 datasets, where random CTCF regions equivalent to ∼1%, 2%, 5% or 10% of JQ1 regions were computationally introduced as true positives of differential binding sites, the identification rate was 95.3%, 98.5%, 99.8% and 99.7%, respectively.
Fig. 12. Genome-wide occupancy of CDK9 and effects of the CDK inhibitor AT7519 on gene transcription. A. Occupancy of CDK9, BRD4, H3K27Ac, MED11 and RNA Pol II at the CCND2 gene locus in MM1.S cells as determined by ChIP-Seq analysis. B. RNA Polymerase II occupancy at the cyclin D2 (CCND2) gene. MM1.S cells were treated with either DMSO (blue) or 2 □M AT7519 (red) for 6 h, followed by RNA Pol II ChIP-Seq analysis. C. RNA Polymerase II traveling ratio distributions in MM1.S cells treated with DMSO (blue) or 2 □M AT7519 (red).
Fig. 13. Genome-wide binding profile of the protein kinase inhibitor AT7519 revealed by Chem-Seq. A. Chemical structures of the pan-CDK inhibitor, AT7519, and its biotinylated version, bio-AT7519. B. Effect of AT7519 (left panel) and its biotinylated version (bio-AT7519, right panel) on MM1.S cell proliferation. Cells were treated with varying concentrations of drug for 72 h as indicated. C. Gene tracks showing occupancy of bio-AT7519 (red) and DMSO (vehicle, blue) in vitro Chem-Seq and CDK9 ChIP-Seq analysis (magenta) at the PRCC gene locus. D. Correlation between bio-AT7519 Chem-Seq signals and CDK9 ChIP-Seq signals (left panel, Spearman correlation: r = 0.785). In contrast, no correlation (r = 0.472) was observed between bio-AT7519 Chem-Seq signals and the ChIP-Seq signals derived from a protein not related to CDK9 (CTCF, right panel).

### DETAILED DESCRIPTION OF THE INVENTION

Therapeutic entities elicit their effects by binding specific proteins or other molecules; some of these target molecules are associated with the genome. Protein components of chromatin biology are emerging as pressing therapeutic targets in cancer, inflammation and cardiovascular disease. Indeed, cancer sequencing efforts have identified numerous mutated, rearranged or amplified chromatin-associated factors and transcription factors, prompting coordinated efforts in chromatin drug discovery world-wide. It has not been possible to determine directly how drugs interact with proteins or other molecular entities that are associated with the genome. While it is possible to use mass spectroscopy to identify the proteins that are bound by small molecule chemicals, this approach does not identify the sites throughout the genome that are occupied by such chemical-bound proteins. While it is possible to use ChIP-seq to identify the sites bound by specific proteins throughout the genome, this approach does not reveal whether these proteins are bound by a molecule of interest.

The development of a method that identifies the sites throughout the genome that are bound by molecules (*e*.*g*., compounds, drugs), directly and/or indirectly, is described herein. Effectively, these data provide molecular resolution on the sites of compound (*e*.*g*., therapeutic) action on the genome, providing spatial target resolution. This information is valuable for *e*.*g*., determining the targets of a potential therapeutic chemical, the sites (genes) where a chemical acts throughout the genome, for assessing specificity of the chemical, and/or for identifying biomarkers for disease and therapy.

A broad range of nucleic acids (*e*.*g*., DNA; RNA) interacting ligands, which are known to bind to the nucleic acids either directly or indirectly (via their target proteins), can be used as affinity 'molecules' to enrich for DNA or RNA derived from cells, tissues or other biological materials.

In some aspects, the method comprises one or more of the following steps shown in Fig.1, which can be performed in the order provided below or in an alternative order. Cellular proteins or protein complexes are linked (e.g., covalently or non-covalently) to cellular nucleic acids (*e.g*., DNA or RNA). In embodiments where the cellular proteins or protein complexes are covalently linked, the linking can be achieved by, for example, treatment with a chemical cross-linker, such as formaldehyde. Cross-linking is achieved either in vivo, in cells, or in vitro (Fig. 1). Cross-linked biological samples are solubilized and diluted in chemical buffers. The nucleic acids contained in the solubilized cross-linked biological samples are fragmented. This can be achieved, for example, by timely controlled sonication (Fig. 1). Modified drugs, enzyme inhibitors and other chemical or natural small molecule compounds are chemically linked (*e*.*g*., covalently; non-covalently) to beads or other entities that enable separation from other materials. The obtained compound-bound beads/entities are added to biological fluids or biological samples, mixed and incubated (Fig. 1). Following incubation in biological samples, the said compound-bound beads/entities are collected and purified. Following purification, nucleic acids are eluted from the said compound-bound beads/entities. Following elution, nucleic acids are purified, concentrated and detected. Detection can be achieved, for example, by sequencing, quantitative polymerase chain reaction or other quantitative methods. If sequenced, the obtained sequencing results reveal the binding sites to which the drug or drug candidate, either directly or indirectly (*e*.*g*., via the said drug target proteins), binds (Fig. 2).

In methods that include a cross linking step, the drug or drug candidate can be added subsequent to the cross-linking, or in the alternative, the method can comprise *e.g*., using cells (*e.g*., live cells or lysates thereof) treated with drug or drug candidates or compounds (*e.g*., using cell permeable compounds or liposome-containing compounds, or other methods of introducing compounds into cells) prior to cross-linking. Another application is to use tissue sections with healthy or diseased cells.

Accordingly, in one aspect, provided herein are methods as defined in the appended claims. In some aspects, the drug or drug candidate can interact with the genome directly. In other aspects, the drug or drug candidate can interact with the genome indirectly, *e*.*g*., the drug or drug candidate interacts with one or more factors that associates with the genome.

In the methods, the genome is contacted with a drug or drug candidate of interest (a drug or drug candidate to be assessed), thereby producing a combination. In particular aspects, the drug or drug candidate is contacted with a genome that is present in a cell and/or cell lysate. The combination is maintained under conditions in which the drug or drug candidate can interact with the genome directly and/or indirectly *e*.*g*., by interacting one or more factors that associate with the genome (*e.g*., of the cell, cell lysate, or nuclear extract that comprises chromatin). The genome is fragmented, thereby producing a mixture comprising genome fragments, wherein one or more of the genome fragments are associated with drug or drug candidate directly and/or indirectly. The sequence of all or a portion of the one or more genome fragments that are associated directly or indirectly with the drug or drug candidate is then determined, thereby mapping, across the genome, the interaction of the drug or drug candidate with the genome.

In a particular aspect, the invention is directed to a method of mapping, across a genome, interaction of a drug directly or indirectly with one or more regions of the genome as defined in the appended claims.

As used herein "mapping the interaction of a cell, cell lysate, or nuclear extract that comprises chromatin across a genome" refers to the identification of the regions of a genome to which a particular drug or drug candidate interacts either directly or indirectly (*e.g.*, *in vivo*; *in vitro*). The regions identified can include a target site of the drug or drug candidate (a site of the genome at which the drug is expected or intended to interact) as well as an off-target site (a site of the genome at which the drug or drug candidate is not expected or intended to interact).

In another aspect, the invention is directed to a method of identifying one or more sites on a genome with which a drug interacts directly or indirectly as defined in the appended claims.

In another aspect, the invention is directed to a method of identifying a signature of a disease across a genome as defined in the appended claims.

In yet another aspect, the invention is directed to a method of obtaining a genetic signature of a drug's interaction with the genome as defined in the appended claims.

Typically, factors that tightly associate with the genome (*e*.*g*., histone) remain bound under the assay conditions described herein; however, factors which do not associate tightly with the genome may not remain bound under the assay conditions described herein. Thus, as described herein, the methods can further comprise covalently linking the one or more factors that associate with the genome to the genome. The one or more factors can be covalently linked to the genome either prior to contacting the genome with the drug or drug candidate or subsequent to contacting the genome with the drug or drug candidate. In one aspect, the cell, cell lysate, or nuclear extract that comprises chromatin can be contacted with the drug or drug candidate after the one or more factors that associate with the genome are covalently linked to the genome. In another aspect, the cell, cell lysate, or nuclear extract that comprises chromatin can be contacted with the drug or drug candidate before the one or more factors are covalently linked to the genome.

Methods for covalently linking one or more factors that associate with the genome of a cell, cell lysate, or nuclear extract that comprises chromatin are known to those of skill in the art. In one aspect, the cell, cell lysate, or nuclear extract that comprises chromatin is contacted with a crosslinking agent such as formaldehyde or paraformaldehyde. In another aspect, the cell, cell lysate, or nuclear extract that comprises chromatin is exposed to UV light (*e*.*g*., with a transilluminator).

As described herein, the methods can further comprise reversing the covalent links (*e*.*g*., the cross links) between the one or more factors and the genome. In some aspects, the covalent links are reversed prior to determining a sequence of all or a portion of the one or more genome fragments that interact with the drug or drug candidate directly and/or indirectly. In other aspects, the covalent links can be reversed after the genome fragments that are associated with one or more factors which interact with the drug or drug candidate are separated from the other genome fragments in the mixture which are not associated with one or more factors which interact with the drug or drug candidate.

Examples of genomes that can be used in the methods of the invention include a prokaryotic genome (*e.g*., a bacterial genome) and a eukaryotic genome (*e.g*., a mammalian genome). In one aspect, the genome is a mammalian (*e.g*., primate, canine, feline, murine and the like) genome. In a particular aspect, the genome is a human genome.

The regions of the genome that can be identified as interacting with a drug or drug candidate include one or more heterochromatic regions of the genome (heterochromatin) and/or one or more euchromatic regions of a genome (euchromatin). In a particular embodiment, the methods provided herein map the interaction of a drug or drug candidate with euchromatin across a genome.

As described herein, the drug or drug candidate can be contacted with a genome that is present in a cell (in vivo Chem-Seq) or cell lysate (in vitro Chem-Seq). Thus, the methods can further comprise obtaining a cell, cell lysate, or nuclear extract that comprises chromatin. Methods of obtaining cells and/or lysates thereof are well known in the art (*e.g*., see Marson et al., Cell, 134(3):521-533 (2008) and US Patent No. 6,410,243 which are incorporated herein by reference). For example, a (one or more) cell can be obtained from an individual (*e*.*g*., a patient) or from commercial sources, *e.g*., a cell and/or tissue bank or storage facility. In particular aspect, cells can be obtained from a sample (*e.g*., a biological fluid (*e.g*., blood, urine, lymph, *etc.*), a tissue sample, a biopsy) from one or more individuals. As will be apparent to those of skill in the art, the cells can be manipulated mechanically and/or chemically (*e*.*g*., by suspending cells in any of a variety of lysis buffers as described herein) to obtain cell lysates.

A variety of cells and lysates thereof can be used herein. Examples of cell and lysates thereof include prokaryotic cells, eukaryotic cells and lysates thereof. In a particular aspect, a mammalian (*e*.*g*., primate, canine, feline, murine, *etc*.) cell or lysate thereof is used. In yet another aspect, a human cell or lysate thereof is used. In still another aspect, a live cell is used. Other aspects of the method comprise use of a normal cell, an abnormal cell and/or lysates thereof. Abnormal cells include diseased cells such as cancer cells, heart disease cells, *etc.* In some embodiments, the cell is fresh, frozen, or preserved (e.g., wherein the cell is a preserved cell from a human sample or sample from a non-human mammal). A sample comprising cells may in some embodiments be obtained from a subject, e.g., a human or a non-human mammal, wherein a drug or drug candidate has been administered to the subject. Cells may be primary cells, immortalized cells, cells of a cell line, genetically modified cells, etc. Cells may be of any cell type or may originate from any organ or tissue in various embodiments. Cells may be, e.g., epithelial cells, hematopoietic cells, immune system cells, nervous system cells, muscle cells, fibroblasts.

The term "drug or drug candidate" refers in some embodiments to a compound that has an activity against (towards) a target of interest (e.g., an IC₅₀ against a target (e.g., a transcription factor) of less than about 1 mM or less than about 100 µM (e.g., less than about 10 µM, less than about 1 µM, or less than about less than about 0.1 µM). As will be appreciated, a compound does not need to be approved by the FDA (or by any government agency responsible for regulating pharmaceuticals in any jurisdiction) to be a drug as used herein. Included herein are compounds (e.g., small molecules) that are active against one or more targets such as a protein (e.g., a protein related to transcription such as a transcription factor). In some embodiments, the drug or drug candidate has been tested and has shown activity in one or more in vitro or in vivo assays. Activity may be, e.g., binding activity (e.g., towards a target), inhibitory or activating activity (e.g., towards a target), activity that alters a cell phenotype or cell state, activity potentially suggesting or indicative that the compound may be of use in treating a disease, etc. Exemplary drug candidates include tool compounds or reference compounds with known activity against a target and compounds identified in an assay such as a screening assay or analogs or derivatives of such compounds, such as analogs or derivatives synthesized, designed, or identified based at least in part on a hit compound in a screen. A drug or drug candidate may modulate, e.g., inhibit or activate, a target of interest. A drug or drug candidate may modulate a target in any of a variety of ways in various embodiments. For example, a drug or drug candidate may bind to a target, may bind to one or more other components that bind to the target, may alter stability or conformation of a target, may affect localization of a target.

In addition, a variety of compounds or drug or drug candidate s can be used in the methods, compositions, kits of the invention. For example, the drug or drug candidate can be a nucleic acid (*e*.*g*., antisense RNA, small interfering RNA (siRNA), a short hairpin RNA (shRNA)), a peptide, a small molecule (*e*.*g*., a small organic chemical), natural produce (e.g., a macrocycle). In a particular aspect, the drug or drug candidate is a therapeutic drug or drug candidate. In another aspect, the drug or drug candidate is a pharmacologically active small molecule. In yet another aspect, the drug or drug candidate is an anti-cancer drug or drug candidate (*e*.*g*., JQ1, tamoxifen). In some embodiments, the drug or drug candidate targets a protein, e.g. inhibits or activates a protein such as a transcription factor or a kinase. In some embodiments, the drug or drug candidate targets DNA or chromatin directly.

The term "small molecule" refers to a compound having a molecular weight of, less than about 7500 amu, less than about 5000 amu, less than about 2500 amu, preferably less than about 2000 amu, even more preferably less than about 1500 amu, still more preferably less than about 1000 amu, or most preferably less than about 750 amu.

As will be appreciated by those of skill in the art, a drug or drug candidate can "interact" with the genome in a variety of ways. For example, a drug or drug candidate can interact with a genome by binding to the genome directly, and/or the drug or drug candidate can interact with a genome indirectly, *e*.*g*., by binding to a factor that is associated with (*e.g*., bound to) the genome.

In still further aspects, the drug or drug candidate can comprise one or more of a variety of detectable labels (*e.g*., a handle) that allow detection and/or separation of the drug or drug candidate at one or more steps of the methods described herein. Such detectable labels are well known to those of skill in the art. Examples of detectable labels include a (one or more) fluorescent molecule, a peptide (*e.g*., avidin, streptavidin), a hapten (*e.g*., dinitrophenyl (DNP), digoxigenin, nitrotyrosine), small molecule (*e*.*g*., biotin) or combinations thereof. Methods for introducing a label onto a drug or drug candidate are well known to those of skill in the art. See, for example, Becer, CR., et al., Angew Chem Int Ed, 48:4900-4908 (2009) and Hein, CD, et al., Pharm Res, 25(10):2216-2230 (2008) which are incorporated herein by reference.

In various embodiments, a drug or drug candidate described herein is attached to a label, for example, through a linker. Exemplary drug or drug candidates described herein are provided in the compound of Formula (I) below: 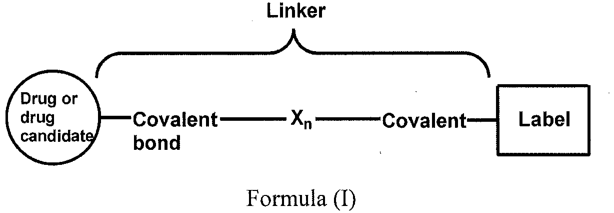 wherein:
Drug or drug or drug candidate is a small molecule
Linker comprise covalent bond-Xn-covalent bond;
each X is independently a covalent bond or a bivalent, straight or branched, saturated or unsaturated, optionally substituted C1-50 hydrocarbon chain wherein one or more methylene units are optionally and independently replaced by - O-, -S-, -Se-, -OP(O)OH-O-, -N(R)P(O)OH-N(R)-, -Si(R)2-, -N(R)-, -C(O)-, - C(O)O-, -OC(O)-, -N(R)C(O)-, -C(O)N(R)-, -(R)NC(O)N(R)-, -(R)C=NN(R)-, - N=N-, -S(O)-, -S(O)2-, -N(R)SO2-, -SO2N(R)-, a haloalkyl group (e.g., -CF2-), a heterocyclic group, an aryl group, or a heteroaryl (e.g., triazole) group;
wherein each occurrence of R is independently hydrogen, a suitable protecting group, or an acyl moiety, arylalkyl moiety, aliphatic moiety, aryl moiety, heteroaryl moiety, or heteroaliphatic moiety;
n is an integer from 1 to 50; and
Label is a moiety that can be used to isolate and/or identify the drug or drug candidate in a method or kit described herein (e.g., fluorescent molecule, a peptide, a hapten or a halo containing compound).

In some embodiments, the linker comprises a polyethylene glycol moiety (PEG) or a derivative thereof (e.g., a PEG 2, PEG 6, PEG 8, PEG 20, PEG 40, PEG 100, PEG 300, PEG 400, PEG 1000).

In some embodiments, the linker comprises a peptide (e.g., an up to 15 amino acid residue peptide).

In some embodiments, the linker comprises a moiety formed using "click chemistry" (e.g., as described in WO 2006/115547). In one embodiment, the linker comprises an amide bond, an ester bond, a disulfide bond, or a triazole.

As described herein, the drug or drug candidate can interact with the genome indirectly *e*.*g*., by interacting with one or more factors that associate with a genome. As used herein, a "factor that associates with the genome" includes a single factor or a combination of factors (a complex of factors such as a transcription complex) that associate with the genome at one or more particular regions. A variety of factors that associate with genomes are well known to those of skill in the art. As is also well known to those of skill in the art, these factors can "associate" with the genome in a variety of ways. For example, a factor can associate with a genome by binding to the genome. Examples of such factors include proteins such as histones, transcription proteins (*e.g*., a transcription regulatory protein or complexes thereof), a protein or complex thereof involved in maintenance of genome integrity (*e*.*g*., a DNA replication and repair protein; a topoisomerase), a chromatin-modifying protein, chromatin associating factor, a receptor (*e*.*g*., a steroid receptor; a nuclear hormone receptor), a kinase, a phosphatase, a protesome, a proteosomal component, a structural or scaffolding factor and complexes thereof. Specific examples are provided in Tables 1-4.

Specific examples of biotinylated compounds for analysis by the Chem-Seq methods described herein are shown in Figure 8, which include BDR inhibitors (BRDi), HDAC inhibitors (HDACi), EZH2 inhibitors (EZH2i), DOT1L inhibitors (DOT1Li) and CDK inhibitors (CDKi). SAHA is a pan-HDAC (HDAC class I and class II) inhibitor, as are related hydroxamic acid HDACi's. FK228 inhibits class I HDACs. Class I includes HDAC1, 2, 3 and 8. Class II (A and B) includes HDAC 4, 5, 6, 7, 9, 10. For the CDKi's, AT7519 is a Cdk9 inhibitor. The PD0332991 compound is a Cdk4/6 inhibitor. The DOT1Li is the bio-PEG version of EPZ004777 from Daigle et al. Cancer Cell 2011 from Epizyme. EZH2 inhibitors are described, *e*.*g*., in WO 2011/140324. Other examples are provided in Table 1.

In the methods provided herein, the cell, cell lysate, or nuclear extract that comprises chromatin is contacted with a drug or drug candidate, thereby producing a combination, and the combination is maintained under conditions in which the drug or drug candidate can interact with the one or more factors that associate with the genome. In the methods which further comprise cross linking the one or more factors associated with the genome to the genome (*e*.*g*., by contacting the cell, cell lysate, or nuclear extract that comprises chromatin with a cross linking agent), the combination is also maintained under conditions in which the one or more factors that associate with the genome are covalently linked to the genome of the cell. In some embodiments, the target is the genome itself. As will be apparent to one of skill in the art, such conditions include appropriate temperature, pH, incubation periods, buffers, *etc.* and will depend upon the assay conditions (*e.g*., the drug or drug candidate being assessed).

The methods provided herein also include a step of fragmenting the genome of the cell, cell lysate, or nuclear extract that comprises chromatin, thereby producing a mixture comprising fragments of the genome (genome fragments), wherein one or more of the fragments are associated with the drug or drug candidate either directly or indirectly. Methods of fragmenting a genome are well known to those of skill in the art. For example, a genome can be fragmented mechanically *e.g*., using sonication and/or shear-inducing methods, and/or chemically *e.g*., using enzyme digestion (*e*.*g*., micrococcal nuclease).

The methods of the invention further comprise determining a (one or more) sequence of all or a portion (an identifying (*e*.*g*., distinct, unique) portion, a portion that allows identification of the sequence within the genome) of the one or more genome fragments that interact with the drug or drug candidate directly and/or indirectly. In a particular aspect, the method comprises determining the sequence of all or a portion of the one or more genome fragments that are associated with the one or more factors which interact with the drug or drug candidate. Methods for determining all or a portion of the sequence that interacts with the drug or drug candidate are well known in the art. Examples of such methods include polymerase chain reaction (PCR), massively parallel sequencing, immobilized nucleic acid polymers, next generation sequencing and the like.

As will be apparent to those of skill in the art, the methods described herein can further comprise isolating one or more (*e*.*g*., all) of the genome fragments that are associated with the drug or drug candidate either directly or indirectly from the mixture of genome fragments (*e*.*g*., separating one or more genomes fragments that are associated with the drug or drug candidate from the genome fragments that are not associated with the drug or drug candidate).

A variety of methods can be used to isolate or separate the one or more genome fragments that are associated with the drug or drug candidate from the mixture of genome fragments. In a particular aspect, such genome fragments are isolated comprising contacting the mixture of genome fragments with an agent that specifically binds to the drug or drug candidate directly and/or indirectly. Examples of agents that can specifically bind to a label (*e.g*., a detectable label) present on the drug or drug candidate include a peptide (*e*.*g*., biotin, avidin, streptavidin), an enzyme (*e.g*., alkaline phosphatase, horseradish peroxidase) and an antibody (*e.g*., a primary antibody, a secondary antibody).

In a particular aspect, the mixture of genome fragments is contacted with a solid support that comprises an agent that specifically binds to the drug or drug candidate directly and/or indirectly. Examples of solid supports for use in the methods include beads, arrays, slides, wells, columns and the like.

The methods described herein can further comprise comparing the results to a suitable control. The controls used in the methods can be a positive and/or negative control. As will be apparent to those of skill in the art, a variety of such controls can be used. An example of a control for use in the methods of mapping, across a genome, interaction of a drug or drug candidate with one or more factors that associate with the genome and/or methods of identifying one or more sites on a genome with which a drug or drug candidate interacts, includes a (one or more) genome map obtained for one or more drug or drug candidates with a known mechanism of action (*e*.*g*., genome maps from a plurality of drug or drug candidates (*e.g*., a class of drug or drug candidates) with a similar mechanism of action; genome maps from a plurality of drug or drug candidates (*e*.*g*., multiple classes of drug or drug candidates) with different (*e*.*g*., a variety of) mechanisms of action). An example of a control for use in the methods of identifying a signature of a disease across a genome includes a signature (*e*.*g*., a known signature) obtained for a similar disease (condition, syndrome) using the same or a similar drug or drug candidate (*e.g*., a drug or drug candidate that has, or is believed to have, a similar mechanism of action). Examples of a control for use in the methods of determining whether a drug or drug candidate will effectively treat a condition in an individual in need thereof include a signature of the drug or drug candidate's interaction across the genome of a cell of lysate thereof obtained from one or more individuals that are or have been treated successfully with the drug or drug candidate (*e*.*g*., a positive control), and/or a signature of the drug or drug candidate's interaction across the genome of a cell of lysate thereof obtained from one or more individuals that are not or have not been treated successfully with the drug or drug candidate (*e*.*g*., a negative control).

### Exemplification

### Example 1 In Vitro Chem-Seq

### Methods/Materials

Biotinylated JQ1 was synthesized from the S enantiomer of JQ-1 referred to herein as (S)-JQ1 or JQ1S. (S)-JQ1 was dissolved in formic acid and stirred at room temperature generated free acid 1. The acid was then coupled with monoprotected PEG-diamine to afford the amide 2. The protecting group (Trt) was removed under acidic condition, and the resulting amine was coupled with biotin to afford final JQ1-PEG-Biotin (*e*.*g*., see Fig. 4; see WO 2011/143669 which is incorporated herein by reference).

Fig. 2 shows the results of differential scanning fluorimetry (DSF) of JQ1-PEG-Biotin bound to Bromodomain 4 (BRD4). The binding of small molecules (*e.g*., JQ1S) to protein (*e.g*., Brd4) will typically generate greater stability of the protein. Therefore, the temperature at which the bound Brd4 protein unfolds will be higher compared to the temperature at which unbound Brd4 protein unfolds. This temperature shift can be measured by the increase of the fluorescence of a dye with affinity for hydrophobic parts of the BRD4 protein. An experiment was conducted by mixing small molecules, dimethyl sulfoxide (DMSO as a control), the S enantiomer of JQ1 (JQ1S), the R enantiomer of JQ1 (JQ1R as a control) and JQ1-Biotin (JQ1-PEG-Biotin) with protein and dye. Due to the unique structure and binding between BRD4 and JQ1, only the S enantiomer is active, and thus, the R enantiomer can be used as a control. Then, using a PCR machine, the solution was slowly warmed up and the temperature at which the protein when bound to each small molecule unfolded was measured. As shown in Figure 2, the data confirmed that JQ1-S and JQ1-Biotin bind to BRD4 to make it more stable and a high temperature was required to unfold BRD4 bound to JQ1S and JQ1-Biotin.

Human SK-MEL-5 cells were grown to 80% confluency with a final count of 1-2x10⁸ cells for each location analysis reaction. Cells were chemically crosslinked by the addition of one-tenth volume of fresh 11% formaldehyde solution for 15 minutes at room temperature. Cells were harvested using a silicon scraper, centrifuged and the derived pellets washed three times with 1xPBS. Cell pellets were flash frozen in liquid nitrogen and stored at -80°C prior to use.

Cells were resuspended, lysed in lysis buffers and sonicated to solubilize and shear crosslinked DNA. Sonication conditions vary depending on cells, culture conditions, crosslinking, equipment, and drug or drug candidate. A Misonix Sonicator 3000 was used and sonication was performed at power 8 for 10 x 30 second pulses (60 second pause between pulses). Samples were kept on ice at all times.

The resulting whole cell extract was incubated overnight at 4°C (affinity purification) with 100 µl of Streptavidin Dynal (MyOne Streptavidin T1) magnetic beads that had been pre-incubated with JQ1 as follows: 5ul of 10mM JQ1/DMSO solution were mixed with 100 ul of Strepavidin Dynal bead suspension and incubated overnight (o.n.). Drug-bound beads were washed four times with BSA blocking solution to remove unbound drug.

The affinity purification was allowed to proceed overnight. Beads were sequentially washed with the following buffers: (1) lysis buffer 1, (2) lysis buffer 2, (3) Sonication buffer, (4) High-salt sonication buffer, (5) LiCl buffer and (6) TE containing 50 mM NaCl. Bound complexes were eluted from the beads by heating at 65°C with occasional vortexing and crosslinking was reversed by overnight incubation at 65°C. Whole cell extract DNA (reserved from the sonication step) was also treated for crosslink reversal.

Affinity-purified DNA and whole cell extract DNA were then treated with RNAse A, proteinase K and purified with multiple phenol:chloroform:isoamyl alcohol extractions.

Purified DNA was prepared for sequencing according to a modified version of the Illumina/Solexa Genomic DNA protocol. Fragmented DNA was prepared for ligation of Solexa linkers by repairing the ends and adding a single adenine nucleotide overhang to allow for directional ligation. A 1:100 dilution of the Adaptor Oligo Mix (Illumina) was used in the ligation step. A subsequent PCR step with limited (18) amplification cycles added additional linker sequence to the fragments to prepare them for annealing to the Genome Analyzer flow-cell. After amplification, a narrow range of fragment sizes was selected by separation on a 2% agarose gel and excision of a band between 150-300 bp (representing shear fragments between 50 and 200nt in length and ∼100bp of primer sequence). The DNA was purified from the agarose and diluted to 10 nM for loading on the flow cell.

The DNA library (2-4 pM) was applied to the flow-cell (8 samples per flow-cell) using the Cluster Station device from Illumina. The concentration of library applied to the flow-cell was calibrated such that polonies generated in the bridge amplification step originated from single strands of DNA. Multiple rounds of amplification reagents were flowed across the cell in the bridge amplification step to generate polonies of approximately 1,000 strands in 1µm diameter spots. Double stranded polonies were visually checked for density and morphology by staining with a 1:5000 dilution of SYBR Green I (Invitrogen) and visualizing with a microscope under fluorescent illumination. Validated flow-cells were stored at 4°C until sequencing.

Flow-cells were removed from storage and subjected to linearization and annealing of sequencing primer on the Cluster Station. Primed flow-cells were loaded into the Illumina Genome Analyzer 1G. After the first base was incorporated in the Sequencing-by-Synthesis reaction the process was paused for a key quality control checkpoint. A small section of each lane was imaged and the average intensity value for all four bases was compared to minimum thresholds. Flow-cells with low first base intensities were re-primed and if signal was not recovered the flow-cell was aborted. Flow-cells with signal intensities meeting the minimum thresholds were resumed and sequenced for 26 cycles.

### Results:

JQ1 is a BET bromodomain inhibitor with potential applications in multiple human cancers (Delmore, J.E. et al., Cell 146, 904-917; Filippakopoulos, P. et al., Nature 468, 1067-1073; Zuber, J. et al., Nature 478, 524-528). It is a competitive Brd2, Brd3, Brd4 and BrdT inhibitor that competes with acetyl-lysine binding. The JQ1 mechanism of action is largely through targeting BET bromodomains, with consequent loss of Myc gene expression, but how it binds target molecules genome-wide is unknown. Using a biotinylated-JQ1, Chem-seq was used to determine where it associates with the genome of SKMEL5 melanoma cells. DMSO was used to determine the background level of beads alone. As shown in Fig. 4, there is a high degree of overlap of the pattern of bio-JQ1 occupancy determined by Chem-Seq with it's the genome-wide occupancy of its protein target Brd4 determined by ChIP-Seq. These results demonstrate that JQ1 binds to Brd4 genome-wide.

Fig. 4 shows chem-seq analysis of the association of bio-JQ1 and genomic regions near the MYC and MITF loci. Enrichment of sequenced reads for DMSO (negative control), bio-JQ1, and Brd4 (a protein target of JQ1) show a high degree of overlap for bio-JQ1 and Brd4 at regions across the genome, including at regions near the MYC (A) and MITF (B) genes.

The method has broad applicability to complement conventional ChIP (which uses antibodies or affinity tags to localize protein:DNA interactions) in cases where ligands for the protein target of interest are available. Importantly, the availability and diversity of these ligands has sharply risen during the past years.

It can be used to reveal the mechanism of drug action as well as the specificity of drugs. Specifically, the method allows a rigorous genome-wide assessment of the specificity and mechanism of small molecule therapeutics, which elicit their effects through binding proteins or nucleic acids where some of the protein targets associate with the genome. The technology described herein allows for the determination of on-target and off-target characterization of drug-genome interactions.

The method is useful for global DNA/RNA-binding studies on entire enzyme and protein families that share a defined mechanism of action. In this case, the function and binding of a whole protein family as such to the genome will be accessible by single analysis using a modified compound targeting the entire protein family. Examples are the genome-wide analyses and thus characterization of the Kinome, HTMome, HDACome etc.

It is useful as a tool in the diagnosis and management of diseases, such as cancer, and thus, can be used in diagnostic laboratories. Drugs have generally a higher stability than antibodies, but, as Fig. 4 shows, are similarly efficient as affinity reagents in enriching for nucleic acids when used according to the herein reported invention, describing the selective enrichment of DNA, a relatively stable molecule which can be detected with high sensitivity.

### Example 2 In Vivo Chem-Seq

The *in vivo* Chem-Seq was carried with the same protocol for *in vitro* Chem-Seq described in Example 1, but differed from the in vitro Chem-Seq protocol in the following steps:
1. Human multiple myeloma (MM1.S) cells (final count of 1-2x10⁸) were treated with 5 uM bio-JQ1 for various incubation times (0, 10, 20, 30 min), followed by addition of fresh formaldehyde solution (final formaldehyde concentration in cell medium is 1%) for 20 min at room temperature. Cells were then washed two times with 1x PBS before cell pellets were flash-frozen in liquid nitrogen and stored at -80 degree prior to use.
2. The nuclear cell extracts (derived from cells treated with compound) were incubated with 200ul of Streptavidin Dynal magnetic beads overnight (o.n.)

Results are shown in Figures 5 and 6. The in vitro method was repeated and compared to the in vivo method (see Figure 7).

### Example 3

### Materials and Methods

### Cell culture and treatment with unbiotinylated drugs

Multiple Myeloma MM1.S cells (CRL-2974, ATCC) were maintained in RPMI-1640 supplemented with 10% fetal bovine serum and 1% GlutaMAX (Invitrogen, 35050-061). For JQ1 treatment experiments, asynchronous cells were treated with varying concentrations of JQ1 or vehicle (DMSO) for 6. Alternatively, cells were treated with 2 µM AT7519 (Selleck Chemicals) for 6h.

### Genome-wide occupancy analysis of drug target proteins (ChIP-Seq)

ChIP coupled with massively parallel DNA sequencing (ChIP-Seq) was performed as previously described (33). The following antibodies were used for Chromatin Immunoprecipitation (ChIP): □nti-BRD4 (Bethyl Labs, A301-985A), □nti-MED1 (Bethyl Labs, A300-793A), □nti-H3K27Ac (Abcam, ab4729), □nti-RNA-Pol II (Santa Cruz, sc-899), □nti-CTCF (Millipore, 07-729) and □nti-CDK9 (sc-484). Illumina sequencing, library construction and ChIP-Seq analysis methods were previously described (33).

### Synthesis of Bio-JQ1(S)

The synthesis of active Bio-JQ1(S) started with the (S)-JQ1, the active enantiomer that inhibits the bromodomain extra-terminal (BET) subfamily. As shown below in Scheme S1, removal of the tert-butyl group of the ester on (S)-JQ produced the acid S1. The resulting acid was then coupled with mono-protected (PEG)2 linked diamine to give an amide S2. The Trt protecting group on the terminal amine of compound S2 was removed under acidic condition to generate free amine, which was further coupled with biotin to afford the final active Bio-JQ1(S). The biotinylated inactive enanitomer Bio-JQ1(R) was synthesized in the same synthetic route using inactive enantiomer (R)-JQ1. See figure 9.

### Expression of recombinant BRD4(1)

The first bromodomain of BRD4 was purified as a poly-histidine-tagged recombinant human protein expressed in E.coli, as previously described (18).

### Differential scanning fluorimetry

Thermal melting experiments were carried out using a 7300 Real Time PCR machine (AB Applied Biosystems). Proteins were buffered in 10 mM HEPES pH 7.5, 500 mM NaCl and assayed in a 96-well plate at a final concentration of 1 µM in 20 µL volume. Compounds were added at a final concentration of 10 µM. SYPRO Orange (Molecular Probes) was added as a fluorescence probe at a dilution of 1:1000. Excitation and emission filters for the SYPRO-Orange dye were set to 465 nm and 590 nm, respectively. The temperature was raised with a step of 4 °C per minute from 25 °C to 96 °C and fluorescence readings were taken at each interval. The observed temperature shifts, ΔTm obs, were recorded as the difference between the transition midpoints of sample and reference (DMSO) wells containing protein without ligand in the same plate.

### Isothermal titration calorimetry

ITC was performed using a ITC200 microcalorimeter from MicroCal™(Northampton, MA). All experiments were carried out at 25 °C while stirring at 1000 rpm, in ITC buffer (50 mM HEPES pH 7.4 at 25 °C, 150 mM NaCl). The microsyringe was loaded with a solution of the protein sample (190 µM, in ITC buffer). All titrations were conducted using an initial injection of 0.2 µl, followed by 19 identical injections of 2 µl with a duration of 5 sec (per injection) and a spacing of 90 sec between injections. The heat of dilution was determined by independent titrations (protein into buffer) and was subtracted from the experimental data. The collected data were implicated in the MicroCal™ Origin software supplied with the instrument to yield enthalpies of binding (ΔH) and binding constants (Ka). A single binding site model was employed. Dissociation constants and thermodynamic parameters are presented in Table 5.

### In vivo genome-wide occupancy analysis of biotinylated JQ1 (In vivo Chem-Seq)

Exponentially growing MM1.S cells (2x108 cells per sample) were treated simultaneously with either 5 uM biotinylated JQ1 (Bio-JQ1) or DMSO (vehicle) and 1% Formaldehyde for 20 min in cell culture medium. Chemical crosslinking was terminated by addition of TRIS buffer, pH 7.5, to a final concentration of 300mM TRIS. Cells were harvested using a silicon scraper, centrifuged and the derived pellets washed three times with PBS. Cell nuclei were prepared as follows: cells were lysed in 50 mM HEPES, pH 7.5, 140 mM NaCl, 1 mM EDTA, 10% Glycerol, 0.5% NP-40, 0.25% Triton X-100 plus protease inhibitor cocktail 'complete' (Roche), and cell nuclei were washed once with 10 mM Tris-HCL, pH 8.0, 200 mM NaCl, 1 mM EDTA, 0.5 mM EGTA and protease inhibitors. Nuclei were resuspended and sonicated in 50 mM Hepes-KOH, pH 7.5, 140 mM NaCl, 1 mM EDTA, 1 mM EGTA, 1% Triton X-100, 0.1% Na-deoxycholate, 0.1% SDS (sonication buffer) and protease inhibitor cocktail at 18 W for 10 cycles (30 s each) on ice with 30 s intervals between cycles. Sonicated lysates were cleared by centrifugation and incubated for 16 - 20 h at 4 °C with magnetic Streptavidin Dynabeads (MyOne Streptavidin T1, Invitrogen) (beads were blocked in PBS containing 0.5% BSA before this incubation step). Following incubation in nuclear sonicated lysate, beads were washed twice in sonication buffer, once in sonication buffer containing 500 mM NaCl, once in LiCl buffer (20 mM Tris-HCL, pH 8.0, 1 mM EDTA, 250 mM LiCl, 0.5% NP-40, 0.5% Na-deoxycholate), and once in 10 mM TRIS, pH 7.5, 0.1 mM EDTA. Bound protein-DNA complexes were subsequently eluted in 50 mM Tris-HCL, pH 8.0, 10 mM EDTA, 10% SDS at 65 °C for 15 min, and crosslinks were reversed by overnight incubation of the eluate at 65 °C. Contaminating RNA and protein were digested by addition of RNase and Proteinase K, respectively, and the DNA purified as previously described (34). Finally, purified DNA fragments were massively parallel sequenced and the sequencing data analyzed as described (33).

### In vitro genome-wide occupancy analysis of biotinylated JQ1 (In vitro Chem-Seq)

Exponentially growing, untreated MM1.S cells were fixed with 1% Formaldehyde for 20 min in cell culture medium. Chemical crosslinking was terminated, cell nuclei prepared and sonicated nuclear lysate obtained as described above. Unlike in the in vivo protocol, however, Streptavidin Dynabeads were pre-incubated in PBS containing 0.5% BSA and either 200 □M biotinylated drug or vehicle (DMSO) for 6 h. Drug-bound beads were subsequently washed four times in PBS/0.5%BSA to remove unbound drug, and incubated in nuclear sonicated lysate for 16 - 20 h at 4 °C. All following steps are identical to those described above (in vivo Chem-Seq method).

### In vitro genome-wide occupancy analysis using biotinylated AT7519 (in vitro Chem-seq)

Exponentially growing, untreated MM1.S cells were fixed with 0.5% Formaldehyde for 5 min in cell culture medium. Chemical crosslinking was terminated by addition of TRIS buffer, pH 7.5, to a final concentration of 300mM TRIS. Cells were washed 3x in PBS and cell nuclei prepared as follows: Cell nuclei were lysed in 50 mM HEPES, pH 7.5, 140 mM NaCl, 1 mM EDTA, 10% Glycerol, 0.5% NP-40, 0.25% Triton X-100 plus protease inhibitor cocktail 'complete' (Roche), and cell nuclei were washed once with 10 mM Tris-HCL, pH 8.0, 200 mM NaCl, 1 mM EDTA, 0.5 mM EGTA and protease inhibitors. Nuclei were resuspended and sonicated in 50 mM Hepes-KOH, pH 7.5, 140 mM NaCl, 1 mM EDTA, 1 mM EGTA, 0.5% NP-40, 0.5% Triton-X (sonication buffer). Pellets were sonicated at 9-12 W for 4 cycles (30 s each) in a Misonix sonicator on ice with 1 min rest intervals between cycles. Drug-bound beads were added to the cleared sonicate and the precipitation allowed to proceed for 12-18 hours. Drug-bound beads were subsequently washed four times in sonication buffer, proteins eluted in 1% SDS, and crosslinks were reversed by overnight incubation of the eluate at 65 °C in 1% SDS. Contaminating RNA and protein were digested by sequential incubation with RNase A and Proteinase K, and the DNA purified as previously described (34). Purified DNA fragments were subjected to massively parallel sequencing (Illumina) and the sequencing data analyzed as described (33).

### Chem-seq and ChIP-Seq data analysis

All ChIP-Seq and Chem-seq datasets were aligned using Bowtie (version 0.12.2) (35) to build version NCBI36/HG18 of the human genome. We used the MACS version 1.4.1 (Model based analysis of ChIP-Seq) (36) peak finding algorithm to identify regions of ChIP-Seq enrichment over background. A p-value threshold of enrichment of 1e-9 was used for all datasets. To obtain the normalized read density of ChIP-Seq datasets in any region, ChIP-Seq reads aligning to the region were extended automatically by MACS and the density of reads per basepair (bp) was calculated. The density of reads in each region was normalized to the total number of million mapped reads producing read density in units of reads per million mapped reads per bp (rpm/bp).

### GEO accession numbers

Aligned and raw data can be found under GEO Accession number GSE43743.

### Definition of transcribed genes

A gene was defined as transcribed if an enriched region for either H3K4me3 or RNA Pol II was located within +/- 5kb of the TSS. H3K4me3 is a histone modification associated with transcription initiation (37).

### Definition of active enhancers

Active enhancers were defined as regions of enrichment for H3K27Ac outside of promoters (greater than 5kb away from any TSS). H3K27Ac is a histone modification associated with active enhancers (38, 39). Active enhancers form loops with promoters that are facilitated by the Mediator complex (25). Thus, H3K27Ac definitions of enhancers were validated using ChIP-Seq data for the mediator subunit Med1. Enriched regions from Med1 had >90% overlap with H3K27Ac regions in all datasets.

### Determination of RNA Pol II traveling ratio

The ratio of RNA Pol II ChIP-Seq levels in initiating to elongating regions a measure known as the traveling ratio (TR), was determined (40). The initiating region was defined as +/-300bp around the TSS. The elongating region was defined as +300bp from the TSS to +3,000bp after the gene end. In order to make higher confidence comparisons, the analysis was limited to genes with detectable signal above noise in the initiating and elongating regions across all samples. The statistical significance of changes in the distribution of traveling ratios was determined using two-tailed t test.

### Quantification of differences in ChIP-seq occupancy at proximal promoters

All genes were ranked by increasing density of RNA Pol II ChIP-Seq reads in the promoter region (+/- 1kb of transcription start site (TSS) and binned in increments of 100 genes. The median ChIP-Seq density for promoter regions within each bin was calculated in rpm/bp for both Pol II, BRD4, MED1, H3K27Ac, CDK9.

### Composite ChIP-seq occupancy profile

Active enhancers were aligned at the center in the composite view of ChIP-Seq density profiles. The average ChIP-Seq read density around +/- 5kb centered on the active enhancer in 50 bp bin was calculated. Genes were aligned using the position and direction of their transcription start sites and transcription termination sites. Data were expressed as genome-wide ChIP-Seq read density averages in 50 bp bins. Correlation between Chem-seq and ChIP-Seq signals were calculated using the enriched regions from targets of small molecules (BRD4 and CDK9) from ChIP-seq. CTCF enriched regions were used as a comparison. Read density profiles of Chem-seq and ChIP-seq were displayed as scatter plots.

### Specificity analysis based on overlap between Chem-Seq and ChIP-Seq data

Chem-Seq and ChIP-Seq data was analyzed to identify genomic regions with substantial JQ1 Chem-Seq signal but no significant BRD4 ChIP-Seq signal, reasoning these sites might represent off-target interactions of JQ1. A generalized linear model (GLM method) was adopted to identify regions with differential signal (41, 42). First identified were the set of genomic regions that were enriched for JQ1 Chem-Seq or BRD4 ChIP-Seq signal in any one of the six datasets being considered (3 replicate JQ1 Chem-Seq datasets and 3 replicate BRD4 ChIP-Seq datasets). Regions from a dataset that overlapped with regions from another data set were merged together to form a representative region that spans the combined genomic region. A total of 25,060 regions were identified. The read density in each region was calculated in units of reads per million mapped reads per bp (rpm/bp) for each dataset. The edgeR package was used to model technical variation due to noise among triplicate datasets and the biological variation due to differences in signal between JQ1 Chem-Seq and BRD4 ChIP-Seq datasets (41). Sequencing depth and TMM techniques were used to normalize datasets after common and tagwise dispersions were estimated. The statistical significance of differences between JQ1 Chem-Seq signals and BRD4 ChIP-Seq signals was calculated using an exact test and resulting P values were subjected to Benjamini-Hochberg multiple testing correction. The analysis identified 10 out of the 25,060 genomic regions examined that showed significant differences in JQ1 Chem-Seq and BRD4 ChIP-Seq signals (FDR<=0.25). All 10 regions showed greater BRD4 ChIP-Seq signal relative to JQ1 Chem-Seq signal; we did not detect any regions showing substantial JQ1 Chem-Seq signal and lacking BRD4 ChIP-Seq signal. These data indicate that, under these experimental conditions, there is no identifiable off-target interaction that results in JQ1 Chem-Seq signal at regions of the genome without BRD4.

To examine the limits of the differential binding analysis described here, simulated datasets that model the presence of JQ1 Chem-Seq signal at regions that were not overlapping with BRD4 ChIP-Seq signals were generated. Then asked was how well the analysis could identify these computationally introduced regions of differential signal. To generate the simulated datasets, each replicate dataset of JQ1 Chem-Seq was spiked with a set of mapped reads from a CTCF ChIP-Seq experiment. The reads mapped specifically to regions of CTCF enrichment, and the selected regions were chosen to avoid overlap with either JQ1 Chem-Seq signal or BRD4 ChIP-Seq signal. Variable numbers of reads were added to simulate the presence of an additional 1%, 2%, 5% or 10% of enriched regions in the JQ1 Chem-Seq datasets. As the CTCF ChIP-Seq reads were not added to BRD4 ChIP-Seq data, regions of CTCF ChIP-Seq-derived signal in the simulated dataset thus served as true positives when testing for the ability to identify differential signal. The GLM-based analysis was then performed as described above. It was found that the GLM analysis identified essentially all regions of differential signal that were introduced in the simulated dataset. In the simulated dataset where an additional 1% of regions were computationally introduced as true positives, 95% of these regions were identified as having differential signal. In the datasets where an additional 2%, 5% or 10% of regions were computationally introduced as true positives, the identification rate was 98.5%, 99.8% and 99.7%, respectively. Together, these data indicate that the GLM method is capable of accurately identifying instances of differential signal, even when they occur rarely.

**TABLE 1**

| **PROTEIN TARGET or CLASS** | **Biotin- DRUG CONJUGATE** | **REGULATORY FAMILY** |
|---|---|---|
| Estrogen receptor | 18b Estradiol | Nuclear Receptors |
| Estrogen receptor | Tamoxifene | Nuclear Receptors |
| Estrogen receptor | Raloxifine | Nuclear Receptors |
| Androgen receptor | Dihydrotestosterone | Nuclear Receptors |
| Androgen receptor | Bicalutamide | Nuclear Receptors |
| Glucocorticoid receptor | Dexamethasone | Nuclear Receptors |
| Retinoic acid receptor | All-trans retinoic acid (ATRA) | Nuclear Receptors |
| Thyroid hormone receptor | Triiodothyronine | Nuclear Receptors |
| Progesterone receptor | Progesterone | Nuclear Receptors |
| Progesterone receptor | Mifepristone | Nuclear Receptors |
| Peroxisome proliferator activated receptor | rosiglitazone | Nuclear Receptors |
| CDK4/6 | PD0332991 | Protein kinases |
| CDK4/6 | LEE011 | Protein kinases |
| CDK7 | THZ | Protein kinases |
| CDK9 | AT7519 | Protein kinases |
| pan-CDK inhibitors | Flavopiridol | Protein kinases |
| pan-protein kinase inhibitor | genistein | Protein kinases |
| DNA interactor | Doxorubicin | DNA |
| DNA interactor | Actinomycin | DNA |
| DNA interactor | Bleomycin | DNA |
| DNA interactor | Etoposide | DNA |
| DNA interactor | Thalidomide | DNA |
| DNA strand cross-linking | Carboplatin | DNA |
| DNA strand cross-linking | Oxaliplatin | DNA |
| EZH2 | EZH2-A | Chromatin regulators |
| EZH2 | EZH2-B | Chromatin regulators |
| BRD2,4 | JQ1 (S) | Chromatin regulators |
| BRD2,4 | JQ1 (R) | Chromatin regulators |
| BRD2,4 | JQ1 (M) | Chromatin regulators |
| BRD2,4 | iBET 151 | Chromatin regulators |
| HDAC | FK228 | Chromatin regulators |
| HDAC | SAHA | Chromatin regulators |
| HDAC | LBH589 | Chromatin regulators |
| HDAC | Valproic Acid | Chromatin regulators |
| Dot1L | EPZ004777 | Chromatin regulators |
| Dot1L | FED1 | Chromatin regulators |
| Dot1L | FED2 | Chromatin regulators |
| PARP | Iniparib | Genomic Integrity |
| PARP | Rucaparib | Genomic Integrity |
| PARP | Veliprib | Genomic Integrity |
| DNA polymerases | Amphidicolin | Genomic Integrity |

**TABLE 2**

| **FACTOR** | **ALIAS** | **UNIPROT ID** | **CLASS** |
|---|---|---|---|
| | | | |
| MLL1 | ALL1, TRX1, KMT2A | Q03164 | WRITER |
| MLL2 | ALR, KMT2B | O14686 | WRITER |
| EZH2 | KMT6 | Q15910 | WRITER |
| Dot1 L | KIAA1814, KMT4 | Q8TEK3 | WRITER |
| MMSET | KIAA1090, WHSC1, NSD2, TRX5 | O96028 | WRITER |
| NSD1 | KMT3B | A4QPE5 | WRITER |
| SETDB1 | KIAA0067, KMT1E | Q15047 | WRITER |
| CBP | CREB-binding protein | Q75MY6 | WRITER |
| SUV39H1/2 | KMT1A, SUV39H | O43463 | WRITER |
| EHMT1 | EUHMTASE1, GLP, KIAA1876, KMT1D | Q9H9B1 | WRITER |
| SMYD2 | KMT3C | Q9NRG4 | WRITER |
| SMYD3 | | Q3B7A0 | WRITER |
| SMYD4 | KIAA1936 | Q8IYR2 | WRITER |
| PRDM1 | BLIMP1, EVI101, PRDI-BF1 | O75626 | WRITER |
| PRDM2 | KMT8, RIZ1 | Q13029 | WRITER |
| PRDM5 | | Q0VAI9 | WRITER |
| PRDM12 | PFM9 | Q9H4Q4 | WRITER |
| PRDM14 | PR domain-containing protein 14 | Q9GZV8 | WRITER |
| PRMT1 | HMT2, HRMT1L2, IR1B4 | Q99873 | WRITER |
| CARM1 | PRMT4 | Q86X55 | WRITER |
| PRMT5 | HRMT1L5, IBP72, JBP1, SKB1 | O14744 | WRITER |
| ASH2L | ASH2L1 | Q9UBL3 | WRITER |
| SETD7 | KIAA1717, KMT7, SET7, SET9 | Q8WTS6 | WRITER |
| G9a | BAT8, C6orf30, KMT1C, NG36, EHMT2 | Q96KQ7 | WRITER |
| SIRT1 | SIR2L1 | Q96EB6 | ERASER |
| HDAC3 | RPD3-2, SMAP45 | O15379 | ERASER |
| HDAC8 | HDACL1, HD8 | Q9BY41 | ERASER |
| HDAC6 | | Q6NT75 | ERASER |
| LSD1 | AOF2, KDM1, KIAA0601, KMDA1 | 060341 | ERASER |
| JARID1A | RBBP2, RBP2, KDM5A | P29375 | ERASER |
| JARID1B | PLU1, RBBP2H1, KDM5B | Q9UGL1 | ERASER |
| JARID1C | DXS1272E,KDM5C, SMCX, XE169 | P41229 | ERASER |
| JMJD3 | KDM6B, KIAA0346 | 015054 | ERASER |
| UTX | KDM6A | O15550 | ERASER |
| BRD4 | HUNK1 | 060885 | READER |
| ING1 | p33; p47; p33ING1; p24ING1c; p33ING1b; p47ING1a | Q9UK53 | READER |
| ING4 | hCG_25927, my036; p29ING4 | Q4VBQ6 | READER |
| ING3 | HSPC301 | Q9NXR8 | READER |
| ING5 | p281NG5 | Q8WYH8 | READER |
| ING2 | ING1L | Q9H160 | READER |
| PHF23 | PDH-containing protein JUNE-1 | Q9BUL5 | READER |
| PYGO2 | PP7910 | Q9BRQ0 | READER |
| ATAD2 | ANCCA | Q6PL18 | READER |
| BRD3 | RING3-like protein, RING3L | Q15059 | READER |
| BRD7 | BP75, CELTIX1 | Q9NPI1 | READER |
| TRIM24 | RNF82, TIF1, TIF1A | O15164 | READER |
| CBX8 | PC3, RC1 | Q9HC52 | READER |
| CBX3 | HP1 gamma | Q13185 | READER |
| WDR5 | BIG3 | P61964 | READER |
| CDK9 | TAK; C-2k; CTK1; CDC2L4; PITALRE | P50750 | KINASES |
| CDK8 | K35 | P49336 | KINASES |
| CDK4 | PSK-J3, Cell division protein kinase 4 | P11802 | KINASES |
| CDK6 | Serine/threonine-protein kinase PLSTIRE | Q00534 | KINASES |
| CDK7 | CAK, CAK1, CDKN7, MO15, STK1 | P50613 | KINASES |
| Aurora A | AURKA, AIK; ARK1; AURA; BTAK; STK6; STK7; STK15; AURORA2; PPP1R47 | O14965 | KINASES |
| SNF5 | RBAF47, INI1, SNF5L1, SMARCB1 | Q12824 | REMODELLERS |
| BAF180 | PBRM1, PB1 | Q86U86 | REMODELLERS |
| ARID1A | BAF250, BAF250A, C1orf4, OSA1, SMARCF1 | 014497 | REMODELLERS |
| BRG1 | BAF190A, SMARCA4, SNF2B, SNF2L4 | P51532 | REMODELLERS |
| TET2 | KIAA1546, Methylcytosine dioxygenase TET2 | Q6N021 | OTHER |
| MENIN | SCG2, MEN1 | O00255 | OTHER |
| ASXL1 | KIAA0978 | Q8IXJ9 | OTHER |
| DNMT1 | AIM, CXXC9, DNMT | P26358 | OTHER |

**TABLE 3**

| **HISTONE MOD** | **ALTERATION AS COMPARED TO NORMAL TISSUE** | **ASSOCIATED CANCER** |
|---|---|---|
| H3K4mel | Decreased | Prostate |
| | Increased Upon Progression | Prostate |
| H3K4me2 | Decreased | Lung, kidney, prostate, non small cell lung carcinoma, hepatocellular carcinoma, breast, panceratic adenocarcinoma |
| | Increased Upon Progression | Lung, kidney, prostate, non small cell lung carcinoma, hepatocellular carcinoma, breast, panceratic adenocarcinoma |
| H3K4me3 | Increased Upon Progression | Prostate cancer |
| | | |
| H3K9me2 | Decreased | Pancreatic adenocarcinomas, prostate, kidney |
| H3K9me3 | Increased | Gastric adenocarcinomas |
| | Decreased | Prostate cancers |
| H3K27me3 | Increased | Paragangliomas |
| | Decreased | Breast, ovarian, pancreatic cancers |
| H3K20me3 | Decreased | Lymphomas, colorectal adenocarcinomas, breast carcinomas |

**TABLE 4**

| **FACTOR** | **ALIAS** | **UNIPROT ID** | **COMPLEX** |
|---|---|---|---|
| WRITERS | | | |
| MLL1 | ALL1, TRX1, KMT2A | Q03164 | MLL1 Complex |
| MLL2 | ALR, KMT2B | O14686 | MLL2/3/ASCOM Complex |
| EZH2 | KMT6 | Q15910 | PRC2 Complex |
| Dot1L | KIAA1814, KMT4 | Q8TEK3 | SEC Complex |
| MMSET | KIAA1090, WHSC1, NSD2, TRX5 | O96028 | |
| NSD1 | KMT3B | A4QPE5 | |
| SETDB1 | KIAA0067, KMT1E | Q15047 | Repressive TRIM28/HP1/MBD1 |
| CBP | CREB-binding protein | Q75MY6 | PCAF SATB1 ING4/5 p/CIP/SRC1 |
| SUV39H1/2 | KMT1A, SUV39H | O43463 | EnoSC complex, HP1 |
| EHMT1 | EUHMTASE1, GLP, KIAA1876, KMT1D | Q9H9B1 | HP1, G9a |
| SMYD2 | KMT3C | Q9NRG4 | Pol II, HELZ, HDAC1 |
| SMYD3 | | Q3B7A0 | |
| SMYD4 | KIAA1936 | Q8IYR2 | |
| PRDM1 | BLIMP1, EVI101, PRDI-BF1 | O75626 | HDAC2, TLE2, LSD1 |
| PRDM2 | KMT8, RIZ1 | Q13029 | |
| PRDM5 | | Q0VAI9 | Methylosome |
| PRDM12 | PFM9 | Q9H4Q4 | |
| PRDM14 | PR domain-containing protein 14 | Q9GZV8 | |
| PRMT1 | HMT2, HRMT1L2, IR1B4 | Q99873 | |
| CARM1 | PRMT4 | Q86X55 | AR, PCAF, NCOA2, P300, NUMAC |
| PRMT5 | HRMT1L5, IBP72, JBP1, SKB1 | 014744 | |
| ASH2L | ASH2L1 | Q9UBL3 | Set1/Ash2/COMPASS complex |

| ERASERS | | | |
|---|---|---|---|
| SIRT1 | SIR2L1 | Q96EB6 | eNOSC, SUV39H1, PCAF, PRC4 |
| HDAC3 | RPD3-2, SMAP45 | 015379 | NCoR/SMRT Complex |
| HDAC8 | HDACL1, HD8 | Q9BY41 | EST1B |
| HDAC6 | | Q6NT75 | |
| LSD1 | AOF2, KDM1, KIAA0601, KMDA1 | O60341 | LSD1/NuRD Complex, REST/RCoR, BHC histone deacetylase complex |
| JARID1A | RBBP2, RBP2, KDM5A | P29375 | |
| JARID1B | PLU1, RBBP2H1, KDM5B | Q9UGL1 | |
| JARID1C | DXS1272E,KDM5C, SMCX, XE169 | P41229 | Part of two distinct complexes, one containing E2F6, and the other containing REST |
| JMJD3 | KDM6B, KIAA0346 | 015054 | MLL4 complex, at least composed of MLL4, ASH2L, RBBP5, WDR5, and KDM6B. |
| UTX | KDM6A | 015550 | Component of the MLL3/MLL4 complex, at least composed of MLL3, MLL4, ASH2L, RBBP5, DPY30, WDR5, NCOA6, KDM6A, PAXIP1/PTIP and C16orf53/PA1. Some fraction of the complex contains MLL2 instead of MLL4 and is named MLL2/MLL3 complex. |

| READERS | | | |
|---|---|---|---|
| BRD4 | HUNK1 | O60885 | pTEFb, SEC |
| ING1 | p33; p47; p33ING1; p24ING1c; p33ING1b; p47ING1a | Q9UK53 | Sin3A/HDAC1 DNMT1 |
| ING4 | hCG_25927, my036; p29ING4 | Q4VBQ6 | TP53 and EP300/p300, HBO1 HAT |
| ING3 | HSPC301 | Q9NXR8 | hNuA4/Tip60 HAT Complex |
| ING5 | p28ING5 | Q8WYH8 | HBO1 or nucleosomal H3-specific MOZ/MORF HATs |
| ING2 | ING1L | Q9H160 | SIN3A, HDAC1, HDAC2, RBBP4/RbAp48, RBBP7/RbAp46, SAP30 |
| PHF23 | PDH-containing protein JUNE-1 | Q9BUL5 | |
| PYGO2 | PP7910 | Q9BRQ0 | BCL9, part of the nuclear beta-catenin/TCF complex |
| ATAD2 | ANCCA | Q6PL18 | Interacts with ESR1 and NCOA3 and these interactions are enhanced by estradio |
| BRD3 | RING3-like protein, RING3L | Q15059 | |
| BRD7 | BP75, CELTIX1 | Q9NPI1 | Interacts with TRIM24, PTPN13 and DVL1. Identified in a complex with SMARCA4/BRG1, SMARCC1/BAF155, SMARCE1/BAF57, DPF2/BAF45D and ARID2, subunits of the SWI/SNF-B (PBAF) chromatin remodeling complex By similarity. Interacts with IRF2 and HNRPUL1. Interacts (via N-terminus) with TP53. Interacts (via C-terminus) with EP300. Interacts with BRCA1. Interacts (via bromo domain) with histone H3 (via N-terminus) acetylated at 'Lys-14' (H3K14ac). Has low affinity for histone H3 acetylated at 'Lys-9' (H3K9ac). Has the highest affinity for histone H3 that is acetylated both at 'Lys-9' (H3K9ac) and at 'Lys-14' (H3K14ac). Has very low affinity for non-acetylated histone H3. Interacts (via bromo domain) with histone H4 (via N-terminus) acetylated at 'Lys-8' (H3K8ac) (in vitro) |
| TRIM24 | RNF82, TIF1, TIF1A | O15164 | Interacts with CARM1, NCOA2/GRIP1, PML, KAT5/TIP60, BRD7, CBX1, CBX3 and CBX5. Part of a coactivator complex containing TRIM24, NCOA2 and CARM1 By similarity. Interacts with NR3C2/MCR. Interacts with the ligand-binding domain of estrogen receptors (in vitro). Interaction with DNA-bound estrogen receptors requires the presence of estradiol. Interacts with AR and p53/TP53. Interacts (via bromo domain) with histone H3 (via N-terminus), provided that it is not methylated at 'Lys-4' (H3K4me0). Does not interact with histone H3 that is methylated at 'Lys-4' (H3K4me1, H3K4me2 or H3K4me3). Interacts (via bromo domain) with histone H3 (via N-terminus) that is acetylated at 'Lys-23' (H3K23ac). Has the highest affinity for histone H3 that is both unmodified at 'Lys-4' (H3K4me0) and acetylated at 'Lys-23' (H3K23ac). Has very low affinity for histone H3 that is methylated at 'Lys-9' (H3K9me), or acetylated at both 'Lys-9' (H3K9ac) and 'Lys-14' (H3K14ac), or acetylated at 'Lys-27' (H3K27ac) (in vitro) |
| CBX8 | PC3, RC1 | Q9HC52 | Component of a PRC1-like complex. Interacts with RING1 RNF2, PCGF1, PCGF2, PCGF3, BMI1, PCGF5 AND PCGF6. Interacts with MLLT3 and histone H3. Interacts with PHC2 |
| CBX3 | HP1 gamma | Q13185 | Binds directly to CHAF1A. Interacts with histone H3 methylated at 'Lys-9'. Part of the E2F6.com-1 complex in G0 phase composed of E2F6, MGA, MAX, TFDP1, CBX3, BAT8, EUHMTASE1, RING1, RNF2, MBLR, L3MBTL2 and YAF2. Interacts with LBR, INCENP, TRIM28/TIF1B, SUV420H1, SUV420H2 and SP100. Interacts with TIF1A |

| KINASES | | | |
|---|---|---|---|
| CDK9 | TAK; C-2k; CTK1; CDC2L4; PITALRE | P50750 | CDK9/cyclin-T (pTEFb), SEC, HEXIM1, HEXIM2, LARP7, BCDIN3, SART3 proteins and 7SK and U6 snRNAs. This inactive 7SK snRNP complex can also interact with NCOR1 and HDAC3, probably to regulate CDK9 acetylation. |
| CDK8 | K35 | P49336 | Mediator complex, which is composed of MED1, MED4, MED6, MED7, MED8, MED9, MED10, MED11, MED12, MED13, MED13L, MED14, MED15, MED16, MED17, MED18, MED19, MED20, MED21, MED22, MED23, MED24, MED25, MED26, MED27, MED29, MED30, MED31, CCNC, CDK8 and CDC2L6/CDK11. The MED12, MED13, CCNC and CDK8 subunits form a distinct module termed the CDK8 module. Mediator containing the CDK8 module is less active than Mediator lacking this module in supporting transcriptional activation. |
| CDK7 | CAK, CAK1, CDKN7, MO15, STK1 | P50613 | Associates primarily with cyclin-H (CCNH) and MAT1 to form the CAK complex. CAK can further associate with the core-TFIIH to form the TFIIH basal transcription factor; this complex is sensitive to UV light. The CAK complex binds to p53/TP53 in response to DNA damage. Interacts with CDK2, SF1/NR5A1, PUF60 and PRKCI. |
| Aurora A | AURKA, AIK; ARK1; AURA; BTAK; STK6; STK7; STK15; AURORA2; PPP1R47 | O14965 | Interacts with CPEB1, JTB, TACC1, TPX2, PPP2CA, as well as with the protein phosphatase type 1 (PP1) isoforms PPP1CA, PPP1CB and PPP1CC. Interacts also with its substrates ARHGEF2, BORA, BRCA1, KIF2A, PARD3, and p53/TP53. Interaction with BORA promotes phosphorylation of PLK1. Interacts with PIFO/C1orf88 |

| REMODELLERS | | | |
|---|---|---|---|
| SNF5 | RBAF47, INI1, SNF5L1, SMARCB1 | Q12824 | BAF, PBAF. Component of the BAF (hSWI/SNF) complex, which includes at least actin (ACTB), ARID1A, ARID1B/BAF250, SMARCA2, SMARCA4/BRG1/BAF190A, ACTL6A/BAF53, ACTL6B/BAF53B, SMARCE1/BAF57 SMARCC1/BAF155, SMARCC2/BAF170, SMARCB1/SNF5/INI1, and one or more of SMARCD1/BAF60A, SMARCD2/BAF60B, or SMARCD3/BAF60C. |
| BAF180 | PBRM1, PB1 | Q86U86 | PBAF. Component of the SWI/SNF-B (PBAF) chromatin-remodeling complex, which contains at least SMARCA4/BRG1, SMARCB1/SNF5/INI1/BAF47, ACTL6A/BAF53A or ACTL6B/BAF53B, SMARCE1/BAF57, SMARCD1/BAF60A, SMARCD2/BAF60B, perhaps SMARCD3/BAF60C, SMARCC1/BAF155, SMARCC2/BAF170, PB1/BAF180, ARID2/BAF200, ARID1A/BAF250A or ARID1B/BAF250B and actin. Interacts with PHF10/BAF45A |
| ARID1A | BAF250, BAF250A, C1orf4, OSA1, SMARCF1 | O14497 | Component of SWI/SNF chromatin remodeling complexes, in some of which it can be mutually exclusive with ARID1B/BAF250B. Component of the BAF (SWI/SNF-A) complex, which includes at least actin (ACTB), ARID1A, ARID1B/BAF250, SMARCA2, SMARCA4/BRG1/BAF190A, ACTL6A/BAF53, ACTL6B/BAF53B, SMARCE1/BAF57, SMARCC1/BAF155, SMARCC2/BAF170, SMARCB1/SNF5/INI1, and one or more of SMARCD1/BAF60A, SMARCD2/BAF60B, or SMARCD3/BAF60C. In muscle cells, the BAF complex also contains DPF3. Component of the SWI/SNF-B (PBAF) complex, at least composed of SMARCA4/BRG1/BAF190A, SMARCB1/BAF47, ACTL6A/BAF53A or ACTL6B/BAF53B, SMARCE1/BAF57, SMARCD1/BAF60A, SMARCD2/BAF60B, perhaps SMARCD3/BAF60C, SMARCC1/BAF155, SMARCC2/BAF170, PB1/BAF180, ARID2/BAF200, ARID1A/BAF250A or ARID1B/BAF250B and actin. Component of the SWI/SNF Brm complex, at least composed of SMARCA2/BRM/BAF190B, SMARCB1/BAF47, ACTL6A/BAF53A or ACTL6B/BAF53B, SMARCE1/BAF57, BAF60 (one or more of SMARCD1/BAF60A, SMARCD2/BAF60B, or SMARCD3/BAF60C), SMARCC1/BAF155, SMARCC2/BAF170, ARID1A/BAF250A, SIN3A, HDAC1, HDAC2, and RBAP4. Component of the |
| BRG1 | BAF190A, SMARCA4, SNF2B, SNF2L4 | P51532 | BAF, PBAF. Component of the CREST-BRG1 complex, at least composed of SMARCA4/BRG1/BAF190A, SS18L1/CREST, HDAC1, RB1 and SP1 By similarity. Interacts with NR3C1, PGR, SMARD1, TOPBP1 and ZMIM2/ZIMP7. Component of the BAF complex, which includes at least actin (ACTB), ARID1A, ARID1B/BAF250, SMARCA2, SMARCA4/BRG1/BAF190A, ACTL6A/BAF53, ACTL6B/BAF53B, SMARCE1/BAF57, SMARCC1/BAF155, SMARCC2/BAF170, SMARCB1/SNF5/INI1, IKFZ1, and one or more of SMARCD1/BAF60A, SMARCD2/BAF60B, or SMARCD3/BAF60C. Interacts directly with IKFZ1 in the BAF complex. In muscle cells, the BAF complex also contains DPF3. Component of the BAF53 complex, at least composed of BAF53A, RUVBL1, SMARCA4/BRG1/BAF190A, and TRRAP, which preferentially acetylates histone H4 (and H2A) within nucleosomes. Component of the WINAC complex, at least composed of SMARCA2, SMARCA4, SMARCB1, SMARCC1, SMARCC2, SMARCD1, SMARCE1, ACTL6A, BAZ1B/WSTF, ARID1A, SUPT16H, CHAF1A and TOP2B. Interacts with (via the bromodomain) with TERT; the interaction regulates Wnt-mediated signaling. Component of neural progenitors-specific chromatin remodeling complex (npBAF complex) composed of |

| Other | | | |
|---|---|---|---|
| TET2 | KIAA1546, Methylcytosine dioxyqenase TET2 | Q6N021 | |
| MENIN | SCG2, MEN1 | O00255 | Component of MLL-containing complexes (named MLL, ASCOM, MLL2/MLL3 or MLL3/MLL4 complex): at least composed ASH2L, RBBP5, DPY30, WDR5, one or several histone methyltransferases (MLL, MLL2, MLL3 and/or MLL4), and the facultative components MEN1, HCFC1, HCFC2, NCOA6, KDM6A, PAXIP1/PTIP and C16orf53/PA1. Interacts with POLR2A phosphorylated at 'Ser-5', but not with the unphosphorylated, nor 'Ser-2' phosphorylated POLR2A forms. Interacts with FANCD2 and DBF4. Interacts with JUND. Interacts with SMAD3, but not with SMAD2, nor SMAD4. Directly interacts with NFKB1, NFKB2 and RELA. |
| ASXL1 | KIAA0978 | Q8IXJ9 | PcG and TrxG. Component of the PR-DUB complex, at least composed of BAP1 and ASXL1. |
| DNMT1 | AIM, CXXC9, DNMT | P26358 | Binds to CSNK1D By similarity. Homodimer. Interacts with HDAC1 and with PCNA. Forms a complex with DMAP1 and HDAC2, with direct interaction. Forms also a stable complex with E2F1, BB1 and HDAC1. Binds MBD2 and MBD3. Component of complexes containing SUV39H1. Interacts with DNMT3A and DNMT3B. Interacts with the PRC2/EED-EZH2 complex. Interacts with UBC9 and BAZ2A/TIP5. |

**Table 5. Shown here are the thermodynamic parameters and dissociation constant (K_{D}) of the binding of biotinylated JQ1 (bio-JQ1) to BRD4's first bromodomain, BRD4(1).**

| Parameters | Value bio-JQ1 |
|---|---|
| N | 1.38±0.0148 |
| K_{D} | 109 ±27.5 M⁻⁹ |
| ΔH | -7159±117.8 cal/mol |
| ΔS | 7.85 cal/mol/deg |

Full references
1. O. Bell, V. K. Tiwari, N. H. Thoma, D. Schubeler, Nat Rev Genet 12, 554 (Aug, 2011).
2. D. M. Gilbert, Nat Rev Genet 11, 673 (Oct, 2010).
3. V. W. Zhou, A. Goren, B. E. Bernstein, Nat Rev Genet 12, 7 (Jan, 2011).
4. R. D. Hawkins, G. C. Hon, B. Ren, Nat Rev Genet 11, 476 (Jul, 2010).
5. K. L. MacQuarrie, A. P. Fong, R. H. Morse, S. J. Tapscott, Trends Genet 27, 141 (Apr, 2011).
6. S. Neph et al., Cell 150, 1274 (Sep 14, 2012).
7. S. H. Orkin, K. Hochedlinger, Cell 145, 835 (Jun 10, 2011).
8. R. A. Young, Cell 144, 940 (Mar 18, 2011).
9. D. L. Northrup, K. Zhao, Immunity 34, 830 (Jun 24, 2011).
10. H. H. Ng, M. A. Surani, Nat Cell Biol 13, 490 (May, 2011).
11. J. S. You, P. A. Jones, Cancer Cell 22, 9 (Jul 10, 2012).
12. J. Sandoval, M. Esteller, Curr Opin Genet Dev 22, 50 (Feb, 2012).
13. C. H. Arrowsmith, C. Bountra, P. V. Fish, K. Lee, M. Schapira, Nat Rev Drug Discov 11, 384 (2012).
14. R. A. Copeland, M. E. Solomon, V. M. Richon, Nat Rev Drug Discov 8, 724 (Sep, 2009).
15. M. A. Dawson, T. Kouzarides, Cell 150, 12 (Jul 6, 2012).
16. A. J. Deshpande, J. Bradner, S. A. Armstrong, Trends Immunol 33, 563 (Nov, 2012).
17. Y. Feng, T. J. Mitchison, A. Bender, D. W. Young, J. A. Tallarico, Nat Rev Drug Discov 8, 567 (Jul, 2009).
18. P. Filippakopoulos et al., Nature 468, 1067 (Dec 23, 2010).
19. E. Nicodeme et al., Nature 468, 1119 (Dec 23, 2010).
20. M. A. Dawson et al., Nature 478, 529 (Oct 27, 2011).
21. C. W. Chung et al., J Med Chem 54, 3827 (Jun 9, 2011).
22. J. E. Delmore et al., Cell 146, 904 (Sep 16, 2011).
23. J. Zuber et al., Nature 478, 524 (Oct 27, 2011).
24. S. Malik, R. G. Roeder, Nat Rev Genet 11, 761 (Nov, 2010).
25. M. H. Kagey et al., Nature 467, 430 (Sep 23, 2010).
26. Y. W. Jiang et al., Proc Natl Acad Sci U S A 95, 8538 (Jul 21, 1998).
27. M. K. Jang et al., Mol Cell 19, 523 (Aug 19, 2005).
28. Z. Yang et al., Mol Cell 19, 535 (Aug 19, 2005).
29. D. Houzelstein et al., Mol Cell Biol 22, 3794 (Jun, 2002).
30. L. Santo et al., Oncogene 29, 2325 (Apr 22, 2010).
31. M. S. Squires et al., Mol Cancer Ther 9, 920 (Apr, 2010).
32. Q. Zhou, T. Li, D. H. Price, Annu Rev Biochem 81, 119 (2012).
33. A. Marson et al., Cell 134, 521 (Aug 8, 2008).
34. T. I. Lee, S. E. Johnstone, R. A. Young, Nat Protoc 1, 729 (2006).
35. B. Langmead, C. Trapnell, M. Pop, S. L. Salzberg, Genome Biol 10, R25 (2009).
36. Y. Zhang et al., Genome Biol 9, R137 (2008).
37. M. G. Guenther, S. S. Levine, L. A. Boyer, R. Jaenisch, R. A. Young, Cell 130, 77 (Jul 13, 2007).
38. A. Rada-Iglesias et al., Nature, (Dec 15, 2010).
39. M. P. Creyghton et al., Proc Natl Acad Sci USA, (Nov 24, 2010).
40. P. B. Rahl et al., Cell 141, 432 (Apr 30, 2010).
41. M. D. Robinson, D. J. McCarthy, G. K. Smyth, Bioinformatics 26, 139 (Jan 1,2010).
42. C. S. Ross-Innes et al., Nature 481, 389 (Jan 19, 2012).

## Claims

1. A method of identifying one or more sites on a genome with which a drug interacts directly or indirectly comprising:
a) contacting a cell or cell lysate with a drug comprising a detectable label;
b) maintaining the cell or cell lysate under conditions in which the drug can interact with one or more genome sites directly or indirectly;
c) fragmenting the genome of the cell or cell lysate that has been contacted with the drug to thereby produce a mixture comprising one or more genome fragments, wherein the one or more genome fragments are directly or indirectly associated with the drug;
d) isolating the one or more genome fragments that are directly or indirectly associated with the drug from the mixture using the detectable label; and
e) determining a sequence of all or a portion of the one or more genome fragments that are directly or indirectly associated with the drug,
thereby identifying one or more sites on the genome with which the drug interacts directly or indirectly.

2. A method of mapping, across a genome, interaction of a drug directly or indirectly with one or more regions of the genome comprising:
a) contacting a cell or cell lysate with a drug comprising a detectable label;
b) maintaining the cell or cell lysate under conditions in which the drug can interact directly or indirectly with the one or more regions of the genome;
c) fragmenting the genome of the cell or cell lysate that has been contacted with the drug to thereby produce a mixture comprising one or more genome fragments, wherein the one or more genome fragments are directly or indirectly associated with the drug;
d) isolating the one or more genome fragments that are directly or indirectly associated with the drug from the mixture using the detectable label; and
e) determining a sequence of all or a portion of the one or more genome fragments that are directly or indirectly associated with the drug,
thereby mapping, across the genome, the interaction of the drug directly or indirectly with the one or more regions of the genome.

3. A method of identifying a signature of a disease across a genome comprising:
a) contacting a cell or cell lysate with a drug, comprising a detectable label, that is used or can potentially be used to treat the disease;
b) maintaining the cell or cell lysate under conditions in which the drug can interact directly or indirectly with one or more regions of the genome;
c) fragmenting the genome of the cell or cell lysate that hs been contacted with the drug to thereby produce a mixture comprising one or more genome fragments, wherein one or more genome fragments are directly or indirectly associated with the drug; and
d) isolating the one or more genome fragments that are directly or indirectly associated with the drug from the mixture using the detectable label; and
e) determining a sequence of all or a portion of the one or more genome fragments that are directly or indirectly associated with the drug,
thereby identifying the signature of the disease across the genome.

4. A method of obtaining a genetic signature of a drug's interaction with the genome comprising:
a) contacting a cell or cell lysate of the individual with a drug comprising a detectable label;
b) maintaining the cell or cell lysate under conditions in which the drug can interact directly or indirectly with one or more regions of the genome;
c) fragmenting the genome of the cell or cell lysate that has been contacted with the drug to thereby produce a mixture comprising one or more genome fragments, wherein the one or more genome fragments are directly or indirectly associated with the drug;
d) isolating the one or more genome fragments that are directly or indirectly associated with the drug from the mixture using the detectable label; and
e) determining a sequence of all or a portion of the one or more genome fragments that are directly or indirectly associated with the drug, thereby obtaining a signature of the drug's interaction across the individual's genome.

5. The method of any one of Claims 1 to 4 wherein the drug interacts indirectly with one or more factors, the method further comprising covalently linking the one or more factors to the genome, thereby producing covalent links between the one or more factors and the genome; optionally wherein (a) the cell or cell lysate is contacted with the drug after the one or more factors that associate with the genome are covalently linked to the genome of the cell or cell lysate; or (b) the cell or cell lysate is contacted with the drug before the one or more factors that associate with the genome are covalently linked to the genome of the cell or cell lysate; or (c) the one or more factors that associate with the genome are covalently linked to the genome of the cell or cell lysate by a method comprising contacting the cell or cell lysate with a crosslinking agent; optionally (i) further comprising terminating crosslinking by the crosslinking agent; optionally wherein the crosslinking is terminated by contacting the cell or cell lysate with a buffer comprising TRIS; or (ii) crosslinking agent is formaldehyde or paraformaldehyde; optionally wherein the formaldehyde is at a concentration of 1% or 0.5%; or (d) further comprising reversing the covalent links prior to determining the sequence of all or a portion of the one or more genome fragments that are associated with the one or more factors which interact with the drug.

6. The method of any one of Claims 1 to 4 wherein the drug comprising the detectable label (a) is a therapeutic drug or a pharmacologically active small molecule; optionally wherein the drug is an anti-cancer drug; or (b) comprises a covalent bond linking the detectable label to the drug; optionally wherein the detectable label is a fluorescent molecule, a peptide, a hapten or a combination thereof; optionally wherein the detectable label is biotin.

7. The method of Claim 5 wherein the one or more factors that associate with the genome is a transcriptional regulatory protein or complex thereof, a protein or complex thereof involved in maintenance of genome integrity or a combination thereof; optionally wherein (a) the transcriptional regulatory protein is a transcription factor, a chromatin-modifying protein, a nuclear hormone receptor, a kinase, a phosphatase, a proteosome, a proteosomal component or a combination thereof; or (b) the protein involved in maintenance of genome integrity is a DNA replication and repair protein, a topoisomerase or a combination thereof.

8. The method of any one of Claims 1 to 4 wherein the genome is fragmented using sonication, shear-inducing methods, enzyme digestion or a combination thereof.

9. The method of any one of Claims 1 to 4 wherein the sequence of the one or more genome fragments that are directly or indirectly associated with the drug is determined using polymerase chain reaction, massively parallel sequencing or a combination thereof.

10. The method of any one of Claims 1 to 4 wherein prior to determining the sequence of the one or more genome fragments that are directly or indirectly associated with the drug, the one or more genome fragments are separated from other genome fragments in the mixture; optionally wherein the one or more genome fragments are separated from other genome fragments in the mixture by contacting the mixture with a solid support that comprises an agent that specifically binds to the drug; optionally wherein (a) the solid support comprises beads; or (b) the agent is an antibody, streptavidin or a combination thereof.

11. The method of any one of Claims 1 to 4 wherein the cell is a live cell or a eukaryotic cell; optionally wherein the eukaryotic cell is a human cell; optionally wherein the cell is a normal cell or an abnormal cell; optionally wherein the abnormal cell is a cancer cell.

12. The method of any one of Claims 1 to 4 wherein one or more sites across the genome associated with the drug directly or indirectly is an off-target site.

13. The method of any one of Claims 1 to 4 wherein the direct or indirect interaction of the drug occurs on one or more euchromatic regions of the genome.

14. The method of Claim 3 further comprising comparing the signature obtained in step e) to a control; optionally wherein the control is a signature of the disease obtained using a drug other than the drug used in step a) that is used to treat the disease.

15. The method of Claim 4 wherein the drug is an anti- cancer drug.

## Patentansprüche

1. Verfahren zur Erkennung einer oder mehrerer Stellen in einem Genom, mit dem ein Wirkstoff direkt oder indirekt wechselwirkt, welches umfasst:
a) Inkontaktbringen einer Zelle oder eines Zell-Lysats mit einem Wirkstoff, der eine erfassbare Markierung umfasst;
b) Erhalten der Zelle oder des Zell-Lysats unter Bedingungen, in denen der Wirkstoff mit einer oder mehrere Genomstellen direkt oder indirekt wechselwirken kann;
c) Fragmentieren des Genoms der Zelle oder des Zell-Lysats, die/das mit dem Wirkstoff in Kontakt gebracht wurde, um ein Gemisch zu erzeugen, das ein oder mehrere Genomfragmente umfasst, wobei das eine oder die mehreren Genomfragmente direkt oder indirekt an den Wirkstoff gebunden sind;
d) Isolieren des einen oder der mehreren Genomfragmente, die direkt oder indirekt an den Wirkstoff gebunden sind von dem Gemisch unter Verwendung der erfassbaren Markierung; und
e) Bestimmen einer Sequenz aller oder eines Bereichs des einen oder der mehreren Genomfragmente, die direkt oder indirekt an den Wirkstoff gebunden sind,
wodurch eine oder mehrere Stellen im Genom, mit denen der Wirkstoff direkt oder indirekt wechselwirkt, erkannt werden.

2. Verfahren zum Kartieren einer Wechselwirkung eines Wirkstoffes direkt oder indirekt mit einer oder mehreren Abschnitten des Genoms über ein Genom, welches umfasst:
a) Inkontaktbringen einer Zelle oder eines Zell-Lysats mit einem Wirkstoff, der eine erfassbare Markierung umfasst;
b) Erhalten der Zelle oder des Zell-Lysats unter Bedingungen, in denen der Wirkstoff direkt oder indirekt mit der einen oder den mehreren Abschnitten des Genoms interagieren kann;
c) Fragmentieren des Genoms der Zelle oder des Zell-Lysats, das mit dem Wirkstoff in Kontakt gebracht wurde, um ein Gemisch zu erzeugen, das das eine oder die mehreren Genomfragmente umfasst, wobei das eine oder die mehreren Genomfragmente direkt oder indirekt an den Wirkstoff gebunden sind;
d) Isolieren des einen oder der mehreren Genomfragmente, die direkt oder indirekt an den Wirkstoff gebunden sind, von dem Gemisch unter Verwendung der erfassbaren Markierung; und
e) Bestimmen einer Sequenz aller oder eines Bereichs des einen oder der mehreren Genomfragmente, die direkt oder indirekt an den Wirkstoff gebunden sind,
wodurch die direkte oder indirekte Wechselwirkung des Wirkstoffes mit der einen oder den mehreren Abschnitten des Genoms im Genom kartiert wird.

3. Verfahren zur Erkennung einer Signatur einer Erkrankung im Genom, welches umfasst:
a) Inkontaktbringen einer Zelle oder eines Zell-Lysats mit einem Wirkstoff, der eine erfassbare Markierung umfasst und der potenziell bei der Behandlung der Erkrankung verwendet wird oder werden kann;
b) Erhalten der Zelle oder des Zell-Lysats unter Bedingungen, in denen der Wirkstoff direkt oder indirekt mit einer oder mehreren Abschnitten des Genoms wechselwirken kann;
c) Fragmentieren des Genoms der Zelle oder des Zell-Lysats, das mit dem Wirkstoff in Kontakt gebracht worden ist, um dadurch eine Gemisch zu erzeugen, das ein oder mehrere Genomfragmente umfasst, wobei das eine oder die mehreren Genomfragmente direkt oder indirekt an den Wirkstoff gebunden sind; und
d) Isolieren des einen oder der mehreren Genomfragmente, die direkt oder indirekt an den Wirkstoff gebunden sind, von dem Gemisch unter Verwendung der erfassbaren Markierung; und
c) Bestimmen einer Sequenz aller oder eines Bereichs des einen oder der mehreren Genomfragmente, die direkt oder indirekt an den Wirkstoff gebunden sind, wodurch die Signatur der Erkrankung im Genom erkannt wird.

4. Verfahren zum Erhalten einer genetischen Signatur einer Wirkstoff-Wechselwirkung mit dem Genom, das umfasst:
a) Inkontaktbringen einer Zelle oder eines Zell-Lysats einer Person mit einem Wirkstoff, der eine erfassbare Markierung umfasst;
b) Erhalten der Zelle oder Zell-Lysats unter Bedingungen, in denen der Wirkstoff direkt oder indirekt mit dem einen oder den mehreren Abschnitten des Genoms wechselwirken kann;
c) Fragmentieren des Genoms der Zelle oder des Zell-Lysats, das mit dem Wirkstoff in Kontakt gebracht wurde, um ein Gemisch zu erzeugen, das eine oder mehrere Genomfragmente umfasst, wobei das eine oder die mehreren Genomfragmente direkt oder indirekt an den Wirkstoff gebunden sind;
d) Isolieren des einen oder der mehreren Genomfragmente, die direkt oder indirekt an den Wirkstoff gebunden sind, von dem Gemisch unter Verwendung der erfassbaren Markierung; und
e) Bestimmen einer Sequenz aller oder eines Bereichs des einen oder der mehreren Genomfragmente, die direkt oder indirekt an den Wirkstoff gebunden sind, wodurch eine Signatur der Wirkstoffs-Wechselwirkung im Genom der Person erhalten wird.

5. Verfahren nach einem der Ansprüche 1 bis 4, wobei der Wirkstoff indirekt mit einem oder mehreren Faktoren wechselwirkt, wobei das Verfahren weiter kovalentes Verbinden des einen oder der mehreren Faktoren mit dem Genom umfasst, wodurch kovalente Verbindungen zwischen dem einen oder den mehreren Faktoren und dem Genom erzeugt werden; wahlweise wobei
(a) die Zelle oder das Zell-Lysat mit dem Wirkstoff in Kontakt gebracht wird, nachdem der eine oder die mehreren Faktoren, die an das Genom gebunden sind, kovalent mit dem Genom der Zelle oder des Zell-Lysats verbunden sind; oder
(b) die Zelle oder das Zell-Lysat mit dem Wirkstoff in Kontakt gebracht worden ist, bevor der eine oder die mehreren Faktoren, die an das Genom binden, mit dem Genom der Zelle oder des Zell-Lysats kovalent verbunden sind; oder
(c) der eine oder die mehreren Faktoren, die an das Genom binden, durch Verfahren kovalent mit dem Genom der Zelle oder des Zell-Lysats verbunden sind, die umfassen: Inkontaktbringen der Zelle oder des Zell-Lysats mit einer vernetzenden Agenz; wahlweise wobei (i) diese weiter ein Beenden des Quervernetzens durch die quervernetzende Agenz umfasst; wahlweise wobei das Vernetzen durch Inkontaktbringen der Zelle oder des Zell-Lysats mit einem TRIS enthaltenden Puffer beendet wird; oder (ii) die vernetzende Agenz Formaldehyd oder Paraformaldehyd ist; wahlweise wobei das Formaldehyd in einer Konzentration von 1% oder 0,5% ist; oder
(d) das Verfahren weiter Auflösen der kovalenten Verbindungen vor dem Bestimmen der Sequenz aller oder eines Bereichs des einen oder der mehreren Genomfragmente umfasst, die an den einen oder die mehreren Faktoren gebunden sind, die mit dem Wirkstoff wechselwirken.

6. Verfahren nach einem der Ansprüche 1 bis 4 wobei der Wirkstoff, der die erfassbare Markierung umfasst,
a) ein therapeutischer Wirkstoff oder eine pharmakologisch wirksame niedermolekulare Substanz ist; wahlweise wobei der Wirkstoff ein Anti-Krebs-Wirkstoff ist; oder
(b) eine kovalente Verbindung umfasst, die die erfassbare Markierung mit dem Wirkstoff verbindet; wahlweise wobei die erfassbare Markierung ein Fluoreszenzmolekül, ein Peptid, ein Hapten oder eine Kombination davon ist; wahlweise wobei die erfassbare Markierung Biotin ist.

7. Verfahren nach Anspruch 5, wobei der eine oder die mehreren Faktoren, die an das Genom binden, ein Transkriptions-regulierendes Protein oder ein Komplex davon, ein Protein oder ein Komplex davon, der an der Erhaltung der Genomintegrität beteiligt ist oder eine Kombination davon sind; wahlweise wobei
(a) das Transkriptions-regulierende Protein ein Transkriptionsfaktor, ein Chromatinmodifizierendes Protein, ein nukleärer Hormonrezeptor, eine Kinase, eine Phosphatase, ein Proteosom, eine protesomale Komponente oder eine Kombination davon ist; oder
(b) das Protein, das an der Erhaltung der Genomintegrität beteiligt ist, ein DNA-Replikations- und Reparaturprotein, eine Topoisomerase oder eine Kombination davon ist.

8. Verfahren nach einem der Ansprüche 1 bis 4, wobei das Genom unter Verwendung von Ultraschall, Scherkraft-auslösenden Verfahren, Enzymverdau oder einer Kombination davon fragmentiert wird.

9. Verfahren nach einem der Ansprüche 1 bis 4, wobei die Sequenz des einen oder der mehreren Genomfragmente, die direkt oder indirekt an den Wirkstoff gebunden sind, unter Verwendung von Polymerase-Ketten-Reaktion, paralleler Massensequenzierung oder einer Kombination davon bestimmt wird.

10. Verfahren nach einem der Ansprüche 1 bis 4, wobei vor dem Bestimmen der Sequenz des einen oder der mehreren Genomfragmente, die direkt oder indirekt an den Wirkstoff gebunden sind, der eine oder die mehreren Genomfragmente von anderen Genomfragmenten in dem Gemisch getrennt werden;
wahlweise wobei das eine oder die mehreren Genomfragmente von anderen Genomfragmenten in dem Gemisch durch Inkontaktbringen des Gemisches mit einem festen Träger getrennt werden, der ein Agenz umfasst, das spezifisch an den Wirkstoff bindet; wahlweise wobei (a) der feste Träger Beads umfasst; oder (b) das Agenz ein Antikörper, Streptavidin oder eine Kombination davon ist.

11. Verfahren nach einem der Ansprüche 1 bis 4, wobei die Zelle eine lebende Zelle ist oder eine eukaryotische Zelle; wahlweise wobei die eukaryotische Zelle eine menschliche Zelle ist; wahlweise wobei die Zelle eine normale Zelle oder eine abnormale Zelle ist; wahlweise wobei die abnormale Zelle eine Krebszelle ist.

12. Verfahren nach einem der Ansprüche 1 bis 4, wobei die eine oder mehreren Stellen in dem Genom, die an den Wirkstoff direkt oder indirekt gebunden sind, *Off-Target-*Stellen sind.

13. Verfahren nach einem der Ansprüche 1 bis 4, wobei die direkte oder indirekte Wechselwirkung des Wirkstoffs an einem oder mehreren euchromatischen Abschnitten des Genoms auftreten.

14. Verfahren nach Anspruch 3, das weiter Vergleichen der in Schritt e) erhaltenen Signatur mit einer Kontrolle umfasst; wahlweise wobei die Kontrolle eine Signatur der Erkrankung ist, die unter Verwendung eines anderen Wirkstoffes als der in Schritt a) verwendete erhalten wurde, der bei der Behandlung der Erkrankung verwendet wird.

15. Verfahren nach Anspruch 4, wobei der Wirkstoff ein Anti-Krebs-Wirkstoff ist.

## Revendications

1. Procédé d'identification d'un ou plusieurs sites sur un génome avec lequel un médicament interagit directement ou indirectement, comprenant :
a) mettre en contact une cellule ou un lysat cellulaire avec un médicament comprenant un marqueur détectable ;
b) maintenir la cellule ou le lysat cellulaire dans des conditions dans lesquelles le médicament peut interagir avec un ou plusieurs sites génomiques directement ou indirectement ;
c) fragmenter le génome de la cellule ou du lysat cellulaire qui a été mis en contact avec le médicament pour produire ainsi un mélange comprenant un ou plusieurs fragments de génome, dans lequel le ou les fragments de génome sont directement ou indirectement associés au médicament ;
d) isoler le ou les fragments de génome qui sont directement ou indirectement associés au médicament à partir du mélange à l'aide du marqueur détectable ; et
e) déterminer une séquence de tout ou partie du ou des fragments de génome qui sont directement ou indirectement associés au médicament,
permettant ainsi d'identifier un ou plusieurs sites sur le génome avec lequel le médicament interagit directement ou indirectement.

2. Procédé de cartographie, à travers un génome, d'une interaction d'un médicament directement ou indirectement avec une ou plusieurs régions du génome comprenant :
a) mettre en contact une cellule ou un lysat cellulaire avec un médicament comprenant un marqueur détectable ;
b) maintenir la cellule ou le lysat cellulaire dans des conditions dans lesquelles le médicament peut interagir directement ou indirectement avec la ou les régions du génome ;
c) fragmenter le génome de la cellule ou du lysat cellulaire qui a été mis en contact avec le médicament pour produire ainsi un mélange comprenant un ou plusieurs fragments génomiques, le ou les fragments génomiques étant directement ou indirectement associés au médicament ;
d) isoler le ou les fragments génomiques qui sont directement ou indirectement associés au médicament à partir du mélange à l'aide du marqueur détectable ; et
e) déterminer une séquence de tout ou partie du ou des fragments de génome qui sont directement ou indirectement associés au médicament,
permettant ainsi de cartographier, à travers le génome, l'interaction du médicament directement ou indirectement avec la ou les régions du génome.

3. Procédé d'identification d'une signature d'une maladie à travers un génome comprenant :
a) mettre en contact une cellule ou un lysat cellulaire avec un médicament, comprenant un marqueur détectable, qui est utilisé ou peut potentiellement être utilisé pour traiter la maladie ;
b) maintenir la cellule ou le lysat cellulaire dans des conditions dans lesquelles le médicament peut interagir directement ou indirectement avec la ou les régions du génome ;
c) fragmenter le génome de la cellule ou du lysat cellulaire qui a été mis en contact avec le médicament pour produire ainsi un mélange comprenant un ou plusieurs fragments de génome, un ou des fragments de génome étant directement ou indirectement associés au médicament ; et
d) isoler le ou les fragments de génome qui sont directement ou indirectement associés au médicament à partir du mélange à l'aide du marqueur détectable ; et
c) déterminer une séquence de tout ou partie du ou des fragments de génome qui sont directement ou indirectement associés au médicament,
permettant ainsi d'identifier la signature de la maladie à travers le génome.

4. Procédé d'obtention d'une signature génétique d'une interaction d'un médicament avec le génome, comprenant :
a) mettre en contact une cellule ou un lysat cellulaire de l'individu avec un médicament comprenant un marqueur détectable ;
b) maintenir la cellule ou le lysat cellulaire dans des conditions dans lesquelles le médicament peut interagir directement ou indirectement avec une ou plusieurs régions du génome ;
c) fragmenter le génome de la cellule ou du lysat cellulaire qui a été mis en contact avec le médicament pour produire ainsi un mélange comprenant un ou plusieurs fragments de génome, dans lequel le ou les fragments de génome sont directement ou indirectement associés au médicament ;
d) isoler le ou les fragments de génome qui sont directement ou indirectement associés au médicament à partir du mélange à l'aide du marqueur détectable ; et
e) déterminer une séquence de tout ou partie du ou des fragments de génome qui sont directement ou indirectement associés au médicament, permettant ainsi d'obtenir une signature de l'interaction du médicament à travers le génome de l'individu.

5. Procédé selon l'une quelconque des revendications 1 à 4, dans lequel le médicament interagit indirectement avec un ou des facteurs, le procédé comprenant en outre la liaison covalente du ou des facteurs au génome, permettant ainsi de produire des liaisons covalentes entre le ou les facteurs et le génome ; facultativement dans lequel (a) la cellule ou le lysat cellulaire est mis en contact avec le médicament après que le ou les facteurs qui s'associent au génome sont liés de façon covalente au génome de la cellule ou du lysat cellulaire ; ou (b) la cellule ou le lysat cellulaire est mis en contact avec le médicament avant que le ou les facteurs qui s'associent au génome sont liés de façon covalente au génome de la cellule ou du lysat cellulaire ; ou (c) le ou les facteurs qui s'associent avec le génome sont liés de façon covalente au génome de la cellule ou du lysat cellulaire par un procédé comprenant la mise en contact de la cellule ou du lysat cellulaire avec un agent réticulant ; facultativement (i) comprenant en outre la terminaison de la réticulation par l'agent réticulant ; facultativement la réticulation étant terminée par mise en contact de la cellule ou du lysat cellulaire avec un tampon comprenant TRIS ; ou (ii) l'agent réticulant est le formaldéhyde ou le paraformaldéhyde ; facultativement le formaldéhyde étant à une concentration de 1 % ou 0,5 % ; ou (d) comprenant en outre l'inversion des liaisons covalentes avant de déterminer la séquence de tout ou partie du ou des fragments de génome qui sont associés au ou aux facteurs qui interagissent avec le médicament.

6. Procédé selon l'une quelconque des revendications 1 à 4, dans lequel le médicament comprenant le marqueur détectable (a) est un médicament thérapeutique ou une petite molécule pharmacologiquement active ; facultativement le médicament étant un médicament anti-cancer ; ou (b) comprend une liaison covalente liant le marqueur détectable au médicament ; facultativement le marqueur détectable étant une molécule fluorescente, un peptide, un haptène ou une combinaison de ceux-ci ; facultativement le marqueur détectable étant la biotine.

7. Procédé selon la revendication 5, dans lequel le ou les facteurs qui s'associent avec le génome sont une protéine régulatrice de transcription ou un complexe de celle-ci, une protéine ou un complexe de celle-ci mis en jeu dans le maintien de l'intégrité du génome ou une combinaison de ceux-ci ; facultativement dans lequel (a) la protéine régulatrice de transcription est un facteur de transcription, une protéine modifiant la chromatine, un récepteur d'hormone nucléaire, une kinase, une phosphatase, un protéosome, un composant protéosomal ou une combinaison de ceux-ci ; ou (b) la protéine mise en jeu dans le maintien de l'intégrité de génome est une protéine de réplication et de réparation de l'ADN, une topoisomérase ou une combinaison de celles-ci.

8. Procédé selon l'une quelconque des revendications 1 à 4, dans lequel le génome est fragmenté à l'aide d'une sonication, de méthodes induisant un cisaillement, d'une digestion enzymatique ou d'une combinaison de celles-ci.

9. Procédé selon l'une quelconque des revendications 1 à 4, dans lequel la séquence du ou des fragments de génome qui sont directement ou indirectement associés au médicament est déterminée à l'aide d'une réaction en chaîne par polymérase, d'un séquençage massivement parallèle ou d'une combinaison de ceux-ci.

10. Procédé selon l'une quelconque des revendications 1 à 4, dans lequel, avant de déterminer la séquence du ou des fragments de génome qui sont directement ou indirectement associés au médicament, le ou les fragments de génome sont séparés des autres fragments de génome dans le mélange ; facultativement dans lequel le ou les fragments de génome sont séparés des autres fragments de génome dans le mélange par mise en contact du mélange avec un support solide qui comprend un agent qui se lie spécifiquement au médicament ; facultativement dans lequel (a) le support solide comprend des billes ; ou (b) l'agent est un anticorps, de la streptavidine ou une combinaison de ceux-ci.

11. Procédé selon l'une quelconque des revendications 1 à 4, dans lequel la cellule est une cellule vivante ou une cellule eucaryote ; facultativement dans lequel la cellule eucaryote est une cellule humaine, facultativement dans lequel la cellule est une cellule normale ou une cellule anormale ; facultativement dans lequel la cellule anormale est une cellule cancéreuse.

12. Procédé selon l'une quelconque des revendications 1 à 4, dans lequel un ou des sites à travers le génome associés au médicament directement ou indirectement est un site hors cible.

13. Procédé selon l'une quelconque des revendications 1 à 4, dans lequel l'interaction directe ou indirecte du médicament a lieu sur une ou plusieurs régions euchromatiques du génome.

14. Procédé selon la revendication 3 comprenant en outre la comparaison de la signature obtenue à l'étape e) avec un témoin ; facultativement dans lequel le témoin est une signature de la maladie obtenue à l'aide d'un médicament autre que le médicament utilisé à l'étape a) qui est utilisé pour traiter la maladie.

15. Procédé selon la revendication 4, dans lequel le médicament est un médicament anti-cancer.
